# EUROPEAN PATENT SPECIFICATION

(11) **EP 1 114 828 B1**
(45) Date of publication and mention of the grant of the patent: **12.03.2008**
(21) Application number: 99943302.2
(22) Date of filing: 10.09.1999
(51) Int. Cl.: C07K 5/075, A23L 1/23, A23L 1/236, C07K 5/06, C07C 229/00

(54) **N-ALKYLASPARTYLDIPEPTIDE ESTER DERIVATIVES AND SWEETENERS**
N-ALKYLASPARTYLDIPEPTIDESTER-DERIVATE UND SÜSSSTOFFE
DERIVES D'ESTER N-ALKYLASPARTYLDIPEPTIDIQUE ET EDULCORANTS

(30) Priority: 18.09.1998 JP 26425298; 16.06.1999 JP 16941999
(43) Date of publication of application: 11.07.2001
(73) Proprietor: Ajinomoto Co., Inc., Tokyo 104-8315 (JP)
(72) Inventor: AMINO, Yusuke, Amino Science Laboratories, Kawasaki-shi, Kanagawa-ken 210-0801 (JP); YUZAWA, Kazuko, Amino Science Laboratories, Kawasaki-shi, Kanagawa-ken 210-0801 (JP); TAKEMOTO, Tadashi, Amino Science Laboratories, Kawasaki-shi, Kanagawa-ken 210-0801 (JP); NAKAMURA, Ryoichiro, Amino Science Laboratories, Kawasaki-shi, Kanagawa-ken 210-0801 (JP)
(74) Representative: Nash, David Allan
(86) International application number: PCT/JP1999/004977
(87) International publication number: WO 2000/017230

(56) References cited:
- EP-A- 0 691 346
- EP-A- 0 784 050
- EP-A- 0 818 463
- WO-A1-99/52937
- GB-A- 1 206 233
- JP-A- 8 503 206
- US-A- 4 645 678
- US-A- 5 480 668

## Description

### Technical Field

This invention relates to a novel N-alkylaspartyl dipeptide ester derivative, a sweetening agent or a sweetened food or the like product comprising the derivative as an effective component.

### Background Art

In recent years, as eating habits have been improved to a high level, fatness caused by excessive sugar intake and diseases accompanied by fatness have been at issue. Accordingly, the development of a low-calory sweetened (sweetening agent) that replaces sugar has been in demand. As a sweetener that has been widely used at present, there is aspartame which is excellent in safety and quality of sweetness US 5480668 and JP 8503206 relate to compounds derived from aspartame for use as sweetening agents.

EP 0784050 relates to aspartylamide derivatives and salts thereof as sweeteners.

EP 0818463 and EP 0691346 relate to the use of aspartyl dipeptide amide derivatives and their salts to provide sweeteners.

US 4645678 relates to compounds derived from aspartame comprising a substituted α-amino group for use as sweeteners.

Gob1206233 relates to aspartic acid dipeptide alkyl esters as sweeteners.

WO 9952937 relates to aspartyl dipeptide ester derivatives as sweeteners.

However, this is somewhat problematic in stability. In the international Patent Publication WO 94/11391, it is stated that derivatives in which an alkyl group is introduced on a nitrogen atom of aspartic acid constituting the aspartame are markedly improved in the sweetening potency and are slightly improved in stability.

Of the compounds stated in this Publication, N-[N-(3,3-dimethylbutyl)-L- α -aspartyl]-L-phenylalanine 1-methyl ester, which has introduced 3,3-dimethylbutyl group as an alkyl group is most excellent. The sweetening potency of this compound is reported to be 10000 times that of sucrose, which is a value obtained on comparing the above compound to a 2%-, 5%- and 10%-sucrose solution. There are also stated aspartame derivatives having introduced 20 types of substituents other than 3,3-dimethylbutyl group. The sweetening potency of these aspartame derivatives is reported to be not higher than 2500 times that of sucrose. There are also stated aspartame derivatives having introduced a 3- (substituted phenyl) propyl group as an alkyl group. Among these, N-[N-(3-phenylpropyl)-L- α-aspartyl] -L-phenylalanine 1-methyl ester and N-[N-(3-(3-methoxy-4-hydroxyphenylpropyl)-L- α - aspartyl]-L-phenylalanine 1-methyl ester, as derivatives having relatively high sweetening potency, are reported to have the sweetening potency of 1500 and 2500 times that of sucrose, respectively. However, the sweetening potency of these derivatives is much lower than that of N-[N-(3,3-dimethylbutyl)-L- α -aspartyl]-L-phenylalanine 1-methyl ester, which is 10000 times that of sucrose. Also, N-[N-[(RS)-3- phenylbutyl]-L- α -aspartyl]-L-phenylalanine 1-methylester, having, as an alkyl group, a substituent corresponding to a 3-phenyl propyl group, to the third position of which a methyl group is further introduced, that is a 3-phenyl butyl group, is reported to have a sweetening potency of 1200 times that of sucrose. It is slightly lowered in sweetening potency in comparison with N-[N-(3-phenylpropyl)-L- α -aspartyl]-L-phenylalanine 1-methyl ester due to the methyl group introduced at the third position. On the other hand, N-[N-[3-(3-methoxy-4-hydroxyphenyl)-(RS)- 1-methylpropyl]-L-α-aspartyll-L-phenylalanine 1-methyl ester, having a structure corresponding to N-[N-[3-(3- methoxy-4-hydroxyphenyl) propyl] -L- α -aspartyl] - L- phenylalanine 1-methyl ester, to the fist position of the propyl group of which a methyl group is introduced, is reported to have a sweetening potency of 500 times that of sucrose. This derivative is lowered significantly in its sweetening potency due to the methyl group introduced at the first position of the propyl group. As an example of substituting the L-phenylalanine methyl ester moiety with another amino acid ester, there is stated N-[N-(3,3-dimethylbutyl)-L- α -aspartyl]-L-tyrosine 1-methyl ester. This derivative is reported to have a sweetening potency of 4000 times that of sucrose. In view of the above-described status of the art, a development of a low-calory sweetening agent having a superior sweetening potency has been requested.

### Problem to be solved by Invention

It is a problem to be solved by the present invention to provide a novel N-alkylaspartyl dipeptide ester derivative having a sweetening potency equivalent or superior to that of the above-described N-[N-[3,3-dimethylbutyl]-L- α -aspartyl]-L phenylalanine 1-methylester, and a low-calory sweetening agent comprising the derivative as an effective component (ingredient).

### Disclosure of Invention

For resolving the above problem, the present inventors have synthesized a variety of compounds in which a variety of 3-(substituted phenyl) propyl group, such as 3,3-dialkyl-3-(substituted phenyl) propyl groups or (RS)-3-alkyl-3-(substituted phenyl) propyl groups, have been introduced on a nitrogen atom of an aspartic acid constituting an aspartame and an aspartame derivative, by reductive alkylation, using a 3-phenylpropionaldehyde derivative, a cinnamaldehyde derivative, a (2-phenylethyl) alkyl ketone derivative or the like having a variety of substituents on a phenyl group and also having 1 to 4 alkyl substituents on the main chain, and examined the sweetening potency of these derivatives. The aspartame derivative is a compound corresponding to the aspartame the L-phenylalanine methyl ester moiety of which is substituted by another amino acid ester therein. As a result of our investigations, the sweetening potency of some of the aspartame derivatives is much higher in sweetening potency than N-[N-(3,3-dimethylbutyl)-L- α -aspartyl]-L phenylalanine 1-methylester reported to have the sweetening potency of 10000 times that of sucrose, to say nothing of N-[N-[(RS)-3-phenylbutyl]-L- α -aspartyl]-L-phenylalanine 1-methylester reported to have the sweetening potency of 1200 times that of sucrose or N-[N-(3,3-dimethylbutyl)-L- α-aspartyl]-L-tyrosine 1-methylester reported to have a sweetening potency equal to 4000 times that of sucrose, as disclosed in the international Patent Publication WO 94/11391, and that, in particular, the compound represented by the general formula (1) below is superior as a sweetening agent. The present invention has been brought to completion based on these findings.

That is, the present invention resides in a N-alkylasparatyl dipeptide ester derivative, inclusive of its salt form, and a sweetening agent or a sweetened food or the like product comprising the derivative, wherein the derivative is represented by the following general formula (1) :

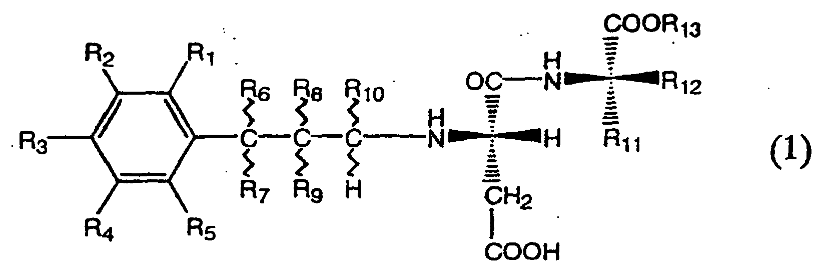

In the above formula, R₁, R₂, R₄ and R₅ are reciprocally independent and denote a substituent selected from the group consisting of a hydrogen atom, a hydroxyl group, an alkoxy group having 1 to 3 carbon atoms, an alkyl group having 1 to 3 carbon atoms and a hydroxy alkyloxy group having two or three carbon atoms
R₃ denotes a substituent selected from the group consisting of a hydroxyl group, an alkoxy group having 1 to 3 carbon atoms, an alkyl group having 1 to 3 carbon atoms and a hydroxy alkyloxy group having two or three carbon atoms;
or R₁ and R₂, or R₂ and R₃ combine together and denote a methylene dioxy group,
provided that in case that R₁ and R₂, or R₂ and R₃ combined together denote a methylene dioxy group, R₄ and R₅ and R₁ or R₃ which is not combined with R₂ together, are reciprocally independent and denote one of the above-mentioned substituents designated for the symbol;
R₆ denotes an alkyl group with 1 to 3 carbon atoms; R₇, R₈, R₉ and R₁₀ are reciprocally independent and denote a substituent selected from the group consisting of a hydrogen atom and an alkyl group with 1 to 3 carbon atoms and optionally two substituents selected from R₆, R₇, R₈, R₉ and R₁₀ therein may combine together and denote an alkylene group with 1 to 5 carbon atoms
provided that, if any optional two substituents selected from R₆, R₇, R₈, R₉ and R₁₀ combine together and denote an alkylene group with 1 to 5 carbon atoms, the substituents other than the selected two are reciprocally independent and denote one of said substituents designated for the symbol;
R₁₁ denotes a substituent selected from the group consisting of a hydrogen atom, a benzyl group, a p-hydroxy benzyl group, a cyclohexyl methyl group, a phenyl group, a cyclohexyl group, a phenyl ethyl group and a cyclohexyl ethyl group, and R₁₂ denotes a substituent selected from the group consisting of a hydrogen atom and an alkyl group with 1 to 3 carbon atoms, and R₁₃ denotes a substituent selected from the group consisting of alkyl groups with 1 to 4 carbon atoms.

In the above formula, linkages denoted by wavy lines are a single linkages, with there being no limitations on the direction of linkage.

If R₆ and R₇, or R₈ and R₉ denote different substituents, or if R₁₀ denotes a substituent other than a hydrogen atom, there is no limitation on the configuration of carbon atoms to which R₆ and R₇ are bonded, those to which R₈ and R₉ are bonded or those to which R₁₀ is linked. For example, these configurations may be (R), (s) or (RS) independently of each other.

### Embodiments for carrying out Invention

The novel N-alkylaspartyl dipeptide ester derivative according to the present invention includes compounds represented by the above formula ,(1) and also salt forms thereof. Of the amino acids constituting the above derivative, the aspartic acrid is in the L-isomer- Other amino acids may be in the L- or D-isomer, as desired.

The compounds of the present invention preferably include the following compounds:
[1] The compounds represented by the above-mentioned formula (1);
   provided that, in the above formula (1), R₁, R₂, R₄ and R₅ are reciprocally independent and denote a substituent selected from the group consisting of a hydrogen atom (H), a hydroxyl group (OH) , an alkoxy group with 1 to 3 carbon atoms (OCH₃, OCH₂CH₃, OCH₂CH₂CH₃ and so forth), an alkyl group with 1 to 3 carbon atoms (CH₃, CH₂CH₃, CH₂CH₂CH₃ and so forth), a hydroxy alkyloxy group with two or three carbon atoms (O(CH₂)₂OH, OCH₂CH (OH) CH₃ and so forth);R₃ denotes a substituent selected from the group consisting of a hydroxyl group (OH), an alkoxy group with 1 to 3 carbon atoms (OCH₃, OCH₂CH₃, OCH₂CH₂CH₃ and so forth), an alkyl group with 1 to 3 carbon atoms (CH₃, CH₂CH₃, CH₂CH₂CH₃ and so forth) and a hydroxy alkyloxy group with two or three carbon atoms (O(CH₂)₂OH, OCH₂CH(OH)CH₃, and so forth); or R₁ and R₂, or R₂ and R₃ therein combine together and denote a methylene dioxy group (OCH₂O).
   It is noted that in case that R₁ and R₂ or R₂ and R₃ combined together denote a methylene dioxy group, R₄ and R₅ and R₁ or R₃ which is not combined with R₂ are reciprocally independent from each other and denote one of the above-mentioned substituents designated respectively for the symbol.
   R₆ denotes an alkyl group with 1 to 3 carbon atoms; R₇, R₈, R₉ and R₁₀ are reciprocally independent from each other and denote a substituent selected from the group consisting of a hydrogen atom and an alkyl group with 1 to 3 carbon atoms and optional two substituents selected from R₆, R₇ R₈, R₉ and R₁₀ combine together and denote an alkylene group with 1 to 5 carbon atoms (such as CH₂, CH₂CH₂, CH₂CH₂CH₂ and so forth).
   In case that optional two substituents selected from the group consisting of R₆ R₇, R₈. R₉ and R₁₀ combined together denote an alkylene group with 1 to 5 carbon atoms, the remaining substituents are reciprocally independent from each other and denote the respective specified or illustrated substituents designated respectively for the symbol
   In the above formula (1), a linkage denoted by wavy line are a single linkage, with there being no limitations on the direction of linkage.
   In case that R₆ and R₇ or R₈ and R₉ denote respective different substituents from each other, or R₁₀ is a substituent other than a hydrogen atom, there is no limitation to the configuration of the carbon atom to which R₆ and R₇ are linked, a carbon atom to which R₈ and R₉ are linked or a carbon atom to which R₁₀ is linked. Thus, the configuration may be any one of (R), (S), (RS) or the like
   R₁₁ denotes a substituent selected from the group consisting of a hydrogen atom, a benzyl group (CH₂C₆H₅) , a p-hydroxybenzyl group (CH₂C₆H₅-p-OH), a cyclohexylmethyl group (CH₂C₆H₁₁), a phenyl group (C₆H₅) , a cyclohexyl group (C₆H₁₁), a phenyl ethyl group (CH₂CH₂C₆H₅) and a cyclohexylethyl group (CH₂CH₂C₆H₁₁), and R₁₂ denotes a substituent selected from the group consisting of a hydrogen atom and an alkyl group with 1 to 3 carbon atoms, and R₁₃ denotes a substituent selected from the group consisting of alkyl groups with 1 to 4 carbon atoms.
[2] The compound as defined in [1] where R₆ is a methyl group.
[3] The compound as defined in [2] where R₇ is a methyl group.
[4] The compound as defined in [3] where R₈ R₉ and R₁₀ are hydrogen atoms.
[5] The compound as defined in [1] to [3] where R₁₀ is a methyl group.
[6] The compound as defined in [1] where R₆ and R₇ combine together and denote an alkylene group having 1 to 5 carbon atoms.
[7] The compound as defined in [1] where R₆ is an alkyl group having two or three carbon atoms
[8] The compound as defined in [1] where any optional two substituents selected from R₆, R₇, R₈, R₉ and R₁₀ combined together denote an alkylene group with one to five carbon atoms.
[9] The derivative in [1] wherein R₃ denotes a methoxy group, R₁, R₂, R₄, R₅, R₇, R₈, R₉, R₁₀ and R₁₂ denote hydrogen atoms, R₆ and R₁₃ denote a methyl group and R₁₁ denotes a benzyl group.
[10] The derivative in [1] wherein R₂ is a methoxy group, R₃ is a hydroxyl group, R₁, R₄, R₅, R₇, R₈, R₉, R₁₀ and R₁₂ denote a hydrogen atom, R₆ and R₁₃ denote a methyl group and R₁₁ denotes a benzyl group.
[11] The derivative in [1] wherein R₂ is a hydroxyl group, R₃ is a methoxy group, R₁, R₄, R_{5,} R₇, R₈, R₉, R₁₀ and R₁₂ denote a hydrogen atom, R₅ and R₁₃ denote a methyl group and R₁₁ denotes a benzyl group
[12] The derivative in [1] wherein R₂ is a methoxy group, R₃ is a hydroxyl group, R₁, R₄, R₅, R₇, R₈, R₉, R₁₀ and R₁₂ denote hydrogen atoms, R₆ and R₁₃ denote a methyl group and R₁₁ denotes a p-hydroxybenzyl group.
[13] The derivative in [1] wherein R₂ is a hydroxyl group, R₃ denotes a methoxy group, R₁, R₄, R₅, R₇, R₈, R₉, R₁₀ and R₁₂ denote a hydrogen atom, R₆ and R₁₃ denote a methyl group and R₁₁ denotes a cyclohexylmethyl group.
[14] The derivative in [1] wherein R₃ is a methoxy group, R₁, R₂, R₄, R₅ R₈, R₉, R₁₀ and R₁₂ denote a hydrogen atom, R₆, R₇ and R₁₃ denote a methyl group, and R₁₁ denotes a benzyl group.
[15] The derivative in [1] wherein R₃ is a hydroxyl group, R₁, R₂, R₄, R₅ ,R₈, R₉, R₁₀ and R₁₂ denote a hydrogen atom, R₅ R₇ and R₁₃ denote a methyl group and R₁₁ denotes a benzyl group.
[16] The derivative in [1] wherein R₂ is a methoxy group, R₃ is a hydroxyl group, R₁, R₄, R₅, R₈, R₉, R₁₀ and R₁₂ denote a hydrogen group, R₆, R₇ and R₁₃ denote a methyl group and R₁₁ denotes a benzyl group.
[17] The derivative in [1] wherein R₂ is a hydroxyl group, R₃ is a methoxy group, R₁, R₄, R₅, R₈, R₉, R₁₀ and R₁₂ denote a hydrogen group, R₆, R₇ and R₁₃ denote a methyl group and R₁₁ denotes a benzyl group.
[18] The derivative in [1] wherein R₂ is a methyl group, R₃ denotes a hydroxyl group, R₁, R₄, R₅, R₇, R₈, R₉ R₁₀ and R₁₂ denote a hydrogen atom, R₆ and R₁₃ denote a methyl group and R₁₁ denotes a benzyl group
[19] The derivative in [1] wherein R₁ is a hydroxyl group, R₃ is a methoxy group, R₂, R₄, R₅, R₈, R₉, R₁₀ and R₁₂denote a hydrogen atom, R₆, R₇ and R₁₃ denote a methyl group and R₁₁ denotes a benzyl group.
[20] The derivative in [1] wherein R₁ is a hydroxyl group, R₃ is a methyl group, R₂, R₄, R₅ R₈, R₉, R₁₀ and R₁₂ denote a hydrogen atom, R₆, R₇ and R₁₃ denote a methyl group and R₁₁ denotes a benzyl group,
[21] The derivative in [1] wherein R₂ and R₃ combine together and denote a methylene dioxy group, R₁, R₄, R₅, R₈, R₉, R₁₀ and R₁₂ denote a hydrogen atom, R₆, R₇ and R₁₃ denote a methyl group and R₁₁ is a benzyl group
[22] The derivative in [1] wherein R₂ denotes a methyl group, R₂ denotes a methoxy group, R₁, R₄, R₅, R₈, R₉, R₁₀ and R₁₂ denote a hydrogen atom, R₆ R₇ and R₁₃ denote a methyl group and R₁₁ denotes a benzyl group.
[23] The derivative in [1] wherein R₂ denotes a methyl group, R₃ is a hydroxyl group, R₁, R₄, R₅, R₈, R₉, R₁₀ and R₁₂ denote a hydrogen atom, R₆, R₇ and R₁₃ denote a methyl group, and R₁₁ denotes a benzyl group.
[24] The derivative in [1] wherein R₂ denotes a hydroxyl group, R₃ denotes a methyl group, R₁, R₄, R₅, R₈ R₉, R₁₀ and R₁₂ denote a hydrogen atom, R₆, R₇ and R₁₃ denote a methyl group and R₁₁ denotes a benzyl group.
[25] The derivative in [1] wherein R₂ denotes a methoxy group, R₃ denotes a hydroxyl group, R₁, R₄, R₅, R₈, R₉, R₁₀ and R₁₂ denote a hydrogen group, R₆ and R₇ combine together and denote a tetramethylene group, R₁₁. denotes a benzyl group and R₁₃ denotes a methyl group.
[26] The derivative in [1] wherein R₂ denotes a hydroxyl group, R₃ denotes a methoxy group, R₁, R₄, R₅, R₈, R₉, R₁₀ and R₁₂ denote a hydrogen atom, R₆ and R₇ denote a methyl group, R₁₁ denotes a benzyl group and R₁₃ denotes an ethyl group.
[27] The derivative in [1] wherein R₂ and R₃ denote a hydroxyl group, R₁, R₄, R₅, R₈, R₉, R₁₀ and R₁₂, denote a hydrogen atom, R₆, R₇ and R₁₃ denote a methyl group, and R₁₁ denotes a benzyl group.
[28] The derivative in [1] wherein R₂ is a hydroxyl group, R₃ denotes a methoxy group, R₁, R₄, R₅, R₈, R₉ and R₁₀ denote a hydrogen atom, R₆, R₇, R₁₂ and R₁₃ denote a methyl group and R₁₁ denotes a benzyl group.
[29] The derivative in [9] to [13] and [18] wherein the configuration of the carbon atom to which R₈ is linked in the formula is in the form of (R), (S) . (RS) or the like.
[30] The derivative in [1] wherein the configuration of the carbon atom to which R₁₀ is linked in the formula is in the form of (R), (S), (RS) or the like.
   For the preferable embodiments, the following inventions are contained in the present invention.
[31] A sweetening agent or a sweetened food or the like product comprising the above-defined derivative in the present invention as an effective component, which may occasionally contain a carrier and/or a bulking agent.
[32] A method for imparting sweetness, comprising a step of: giving (mixing or adding) the above derivative to a product in need of sweetness, such as food, beverage (drinking), pharmaceuticals and oral hygiene products, and products requesting sweetness for animals other than humans.
[33] A method for manufacturing a compound represented by the above general formula (1), where R₁₀ denotes a hydrogen atom, said method comprising a step of reacting an aldehyde shown by the following general formula (2): 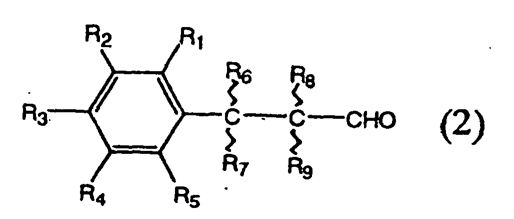 where R₁, R₂, R₃, R₄, R₅, R₆, R₇, R₈ and R₉ have the same meaning as R₁, R₂, R₃, R₄, R₅, R₆, R₇, R₈ and R₉, respectively in the above formula (1);
   It being noted that linkages denoted by wavy lines in the above formula (2) are single linkages, with there being no limitations on the direction of linkage;
   it being also noted that, if R₆ and R₇, or R₈ and R₉ are not the same substituents, there is no particular limitation to the configuration of carbon atoms to which R₆ and R₇, or R₈ and R₉ are linked, such that it may be (R), (S), (RS) or the like whichever is desired;
   with an aspartame derivative shown by the following general formula (3): 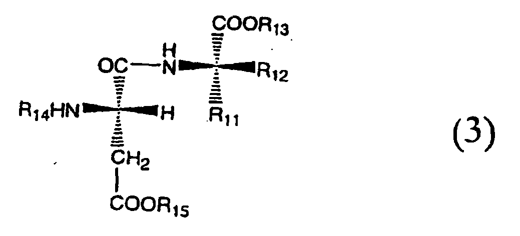 under a reductive alkylation condition;
   wherein R₁₁, R₁₂ and R₁₃ in the above formula (3) have the same meaning as R₁₁, R₁₂ and R₁₃, respectively in the above formula (1), R₁₄ denotes a hydrogen atom or a substituent that can be converted into a hydrogen atom under the reductive alkylation condition and R₁₅ denotes a hydrogen atom, a benzyl group or a substituent that may be used for protecting a carboxyl group such as a t-butyl group or the like.
[34] A method for manufacturing a compound represented by the above general formula (1), where R₇, R₉ and R₁₀ denote a hydrogen atom, said method comprising a step of reacting an aldehyde shown by the following general formula (4) 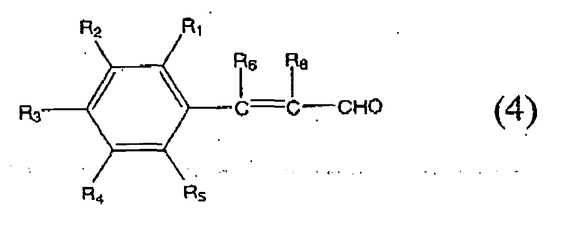 where R₁, R₂, R₃, R₄, R₅, R₆ and R₈ have the same meaning as R₁, R₂, R₃, R₄, R₅, R₆ and R₈, respectively in the above formula (1);
   with an aspartame derivative shown by the above-mentioned general formula (3) under a reductive alkylation condition.
[35] manufacturing compound the above general formula (1), comprising a step of reacting an aldehyde shown by the following general formula (5) 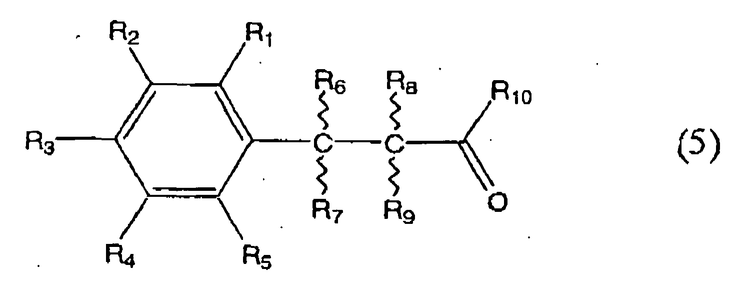
where R₁, R₂, R₃, R₄, R₅, R₆, R₇, R₈, R₉ and R₁₀ have the same meaning as R₁, R₂, R₃, R₄, R₅, R₆, R₇, R₈, R₉ and R₁₀, respectively in the above formula (1);
it being noted that linkages denoted by wavy lines in the above formula (5) are single linkages, with there being no limitations on the direction of linkage;
it being also noted that, if R₆ and, R₇ or R₈ and R₉ are not the same substituents, there is no particular limitation to the configuration of the carbon atoms to which R₆ and R₇, or R₈ and R₉ are linked, such that it may be (R), (S), (RS) or the like whichever is desired;
with the aspartame derivative shown by the above general formula (3) under a reductive alkylation condition.

It is sufficient if the manufacturing methods given in [33] to [35] include the reacting step under the reductive alkylation condition, such that a step or steps other than the reacting step under the reductive alkylation condition may also be included in the manufacturing method. There may also be included an optional step or steps, following the reacting step under the reductive alkylation condition, for example, de-protection in a hydroxyl group or the other functional group, a salt forming step or the like, for producing such targeted compounds.

As the substituent that can be converted into a hydrogen atom under the reductive alkylation condition, those that usually can be used for such purpose, such as benzyloxy carbonyl group or the like, may be optionally selected depending on the particular reductive alkylation condition used. As these reductive alkylation conditions, the conditions as known per se, or any suitable conditions that will be developed in future, such as a condition employing metal hydrides, may be used, as needed.

As a further preferable present embodiment of the present invention, if aldehyde shown by the general formulas (2), (4) or (5) includes hydroxyl groups, the above-described manufacturing methods of [33] to [35] employing an aldehyde the hydroxyl group of which is protected by a suitable protecting group, such as benzyl group, may also be contained in the present invention.

It is noted that salts of the compounds of the present invention, which are included in the derivatives of the present invention, may be enumerated by, for example, salts of alkali metals such as sodium and potassium, salts of alkali earth metals, such as calcium and magnesium, ammonium salt with ammonia, salts with amino acids, such as lysine and arginine, salts with inorganic acids, such as hydrogen chloride and sulfuric acid, salts with organic acids, such as citric acid and acetic acid, and salts with sweetening agents, such as saccharin, acesulfame, cyclamic acid and glycyrrhizic acid. These salts may be included in the derivatives of the present invention, as pointed out above.

The N-alkylaspartyl dipeptide ester derivative of the present invention can be easily synthesized by reductive alkylation of aspartame or aspartame derivatives, that is compounds obtained on replacing an L-phenylalanine methylester moity in the aspartame by another amino acid ester, using a 3-phenylpropionaldehyde derivative, a cinnamaldehyde derivative or a (2-phenylethyl) alkylketone derivative, which has different substituents on a phenyl group and also having one to four alkyl substituents on the main chain, and a reducing agent, such as a hydrogen/palladium carbon catalyst. Alternatively, the N-alkylaspartyl dipeptide ester derivative of the present invention can be produced by a method consisting in reductive alkylation of an aspartame derivative, having a protecting group in a β-position in the carboxylic acid, such as β -O-benzyl- α -L-aspartyl-L-amino acid methyl ester, using the above-described 3-phenylpropionaldehyde derivative, a cinnamaldehyde derivative or a (2-phenylethyl) alkylketone derivative, and a reducing agent, such as NaB(OAc)₃H, as disclosed in A. F. Abdel - Magid et al., Tetrahedron letters, 31, 5595 (1990), followed by removal of protecting groups thereof, or by a method consisting in saturating unsaturated bonds with a reducing agent, as the occasion may demand. The above aspartame derivative may be obtained by a usual peptide synthesis method, as discussed in Izumiya et al., Fundamentals and Experimentation in Peptide Synthesis. Published by MARUZEN on January 20, 1985. The method for synthesis of the compounds in the present invention is, however, not limited to these methods. In place of the above-mentioned 3-phenylpropionaldehyde derivative, cinnamaldehyde derivative or the (2-phenylethyl) alkyl ketone derivative, acetal or ketal derivatives thereof may, of course, be used as the aldehyde or ketone components at the time of the reductive alkylation.

As a result of sensory evaluation, the derivative, that is the compound, and salt forms thereof, according to the present invention, have been found to have strong sweetening potency and sensory properties similar to that of sugar. For example, the sweetness of N-[N-[3-(3-hydroxy-4-methoxyphenyl)-3- methylbutyl]-L- α -aspartyl]-L-phenylalanine 1-methyl ester was approximately 70000 times that of sugar, the sweetness of N-[N-[3-(3-methyl-4-hydroxyphenyl)-3-methylbutyl]-L- α -aspartyl]-L-phenylalanine 1-methyl ester was approximately 70000 times that of sugar, the sweetness of N-[N-[3-(3-hydroxy-4-methylphenyl)-3-methylbutyl]-L- α - aspartyl]-L-phenylalanine 1-methyl ester was approximately 60000 times that of sugar, and the sweetness of N-[N-[(RS)-3-(3-hydroxy-4-methoxyphenyl) butyl]-L- α -aspartyl] -L-phenylalanine 1-methyl ester was approximately 50000 times that of sugar. On the other hand, the half life in a buffer of pH = 3.0 at 72.0 °C of N-[N-[3-(3-methoxy-4-hydroxyphenyl)-3-methylbutyl]-L- α -aspartyl]-L-phenylalanine 1-methyl ester was 34.4 hours, which was substantially equivalent to the half life of N-[N-(3,3-dimethylbutyl)-L- α -aspartyl]-L-phenylalanine 1-methyl ester (31.4 hours under the same condition). Also, the half life in a buffer with pH=3.0 at 70.0°C of aspartame, N-[N-(3,3-dimethylbutyl)-L- α- aspartyl]- L-phenylalanine 1 -methyl ester, N-[N-[3-(3-hydroxy-4-methoxyphenyl)-3-methylbutyl]-L-α-aspartyl]-L-phenylalanine 1- methyl ester and N-[N-[3-(4-hydroxyphenyl)-3-methylbutyl]-L- α-aspartyl]-L-phenylalanine 1-methylester, was measured, and found to be 23.5, 38.3, 44.5 and 43.6 hours, respectively.

Table 1 shows the structures of several synthesized N-alkyl aspartyl dipeptide ester derivatives, shown by the general formula (6), indicated below, and results of the sensory evaluation tests.

As may be seen from the results of Table 1, the novel derivatives of the present invention are particularly excellent in sweetness (sweetening potency). 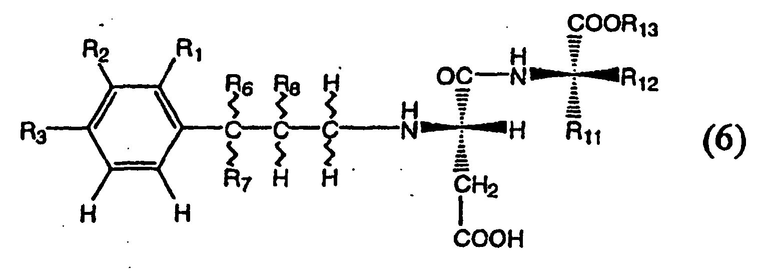 Structure of N-alkylasparatyl dipeptide ester derivative and sweetness potency

**Table 1**

| Compound | | R₂ | R₃ | R₆ | R₇ | R₈ | R₁₁ | R₁₂ | R₁₃ | sweetness potency ^{*)} |
|---|---|---|---|---|---|---|---|---|---|---|
| No. | R₁ | | | | | | | | | |
| 1 | H | H | OCH₃ | CH₃ | H | H | CH₂C₆H₅ | H | CH₃ | 16000 |
| 2^{†} | H | OH | H | CH₃ | H | H | CH₂C₆H₅ | H | CH₃ | 12000 |
| 3 | H | OCH₃ | OH | CH₃ | H | H | CH₂C₆H₅ | H | CH₃ | 30000 |
| 4 | H | OH | OCH₃ | CH₃ | H | H | CH₂C₆H₅ | H | CH₃ | 50000 |
| 5 | H | OCH₃ | OH | CH₃ | H | H | CH₂C₆H₄∩-p-∩OH | | | |
| | | | | | | | | H | CH₃ | 25000 |
| 6 | H | OH | OCH₃ | CH₃ | H | H | CH₂C₆H₁₁ | H | CH₃ | 40000 |
| 7 | H | H | OCH₃ | CH₃ | CH₃ | H | CH₂C₆H₅ | H | CH₃ | 25000 |
| 8 | H | H | OH | CH₃ | CH₃ | H | CH₂C₆H₅ | H | CH₃ | 25000 |
| 9 | H | OCH₃ | OH | CH₃ | CH₃ | H | CH₂C₆H₅ | H | CH₃ | 40000 |
| 10 | H | OH | OCH₃ | CH₃. | CH₃ | H | CH₂C₆H₅ | H | CH₃ | 70000 |
| 11 | H | CH₃ | OH | CH₃ | H | H | CH₂C₆H₅ | H | CH₃ | 50000 |
| 12^{†} | H | OH | OCH₃ | H | H | CH₃ | CH₂C₆H₅ | H | CH₃ | 5000 |
| 13^{†} | OH | H | H | CH₃ | CH₃ | H | CH₂C₆H₅ | H | CH₃ | 8000 |
| 14 | OH | H | OCH₃ | CH₃ | CH₃ | H | CH₂C₆H₅, | H | CH₃ | 20000 |
| 15 | OH | H | CH₃ | CH₃ | CH₃, | H | CH₂C₆H₅ | H | CH₃ | 25000 |
| 16 | H | OCH₂O | | CH₃ | CH₃ | H | CH₂C₆H₅ | H | CH₃ | 30000 |
| 17 | H | CH₃ | OCH₃ | CH₃ | CH₃ | H | CH₂C₆H₆ | H | CH₃ | 30000 |
| 18 | H | CH₃ | OH | CH₃ | CH₃ | H | CH₂C₆H₅ | H | CH₃ | 70000 |
| 19 | H | OH | CH₃ | CH₃ | CH₃ | H | CH₂C₆H₅ | H | CH₃ | 60000 |
| 20 | H | OCH₃ | OH | CH₂CH₂CH₂CH₂ | | H | CH₂C₆H₅ | H | CH₃ | 30000 |
| 21 | H | OH | OCH₃ | CH₃ | CH₃ | H | CH₂C₆H₅ | H | CH₂CH₃ | 15000 |
| 22 | H | OH | OCH₃ | CH₃ | CH₃ | H | CH₂C₆H₅ | CH₃ | CH₃ | 40000 |
| 23 | H | OH | OR | CH₃ | CH₃ | H | CH₂C₆H₅ | H | CH₃ | 50000 |

| | | | | | | | | | | |
|---|---|---|---|---|---|---|---|---|---|---|
| *)values compared to sweetening potency of a 4% aqueous solution of sucrose †) Comparative examples | | | | | | | | | | |

Meanwhile, in case that the derivatives of the present invention (compounds of the present invention inclusive of salt forms thereof) are used as a sweetening agent, it is of course possible to use other sweetening agents in combination.

If the derivatives of the present invention are used as the sweetening agents, it is of course possible to use a carrier and/or a bulking agent, for example, a carrier or a bulking agent so far known and used.

The derivatives of the present invention can be used as a sweetening agent or a component thereof. In addition, the derivatives of the present invention can be used for products, such as foods or the like products, in need of a sweet taste, such as confectionery, chewing gums, hygiene products, toiletries, cosmetics, pharmaceuticals and various veterinary products for animals other than those for humans. Moreover, the derivatives of the present invention can be used as form of a product to be given or endowed with a sweet taste and in a sweet taste imparting method for the products ( foods or the like products ) in need of a sweet taste. As for the method of using the derivatives of the present invention, any suitable conventional or well-known method can be followed.

### Preferred Embodiments for Carrying out Invention

The present invention will be explained in detail with reference to Examples which, however, are merely illustrative and are not intended to limit the present invention.

The NMR spectrum and the MS spectrum were measured using Varian Gemini 300 (300 MHz) and Thermo Quest TSQ700, respectively.

### (Example 1)

### Synthesis of N-[N-[3-(3-hydroxy-4-methoxyphenyl)-3-methylbutyl]-L-α-aspartyl]-L-phenylalanine 1-methyl ester (Table 1, compound number 10)

To 703 mg (1.45 mmol) of N-t-butoxycarbonyl- β -O-benzyl-α-L-aspartyl-L-phenylalanine methyl ester, 10 ml of a 4N-HCl/dioxane solution were added and stirred at room temperature for one hour. The reaction solution was concentrated under reduced pressure. To the residue were added 50 ml of a 5%-aqueous solution of sodium hydrogen carbonate and extraction was made twice with 50 ml of ethyl acetate. An organic layer was washed with a saturated saline water and dried over anhydrous magnesium sulfate. Magnesium sulfate was filtered off and the liquid filtrate was concentrated under reduced pressure to yield 557 mg (1.45 mmol) of β-O-benzyl- α-L-aspartyl-L-phenylalanine methyl ester, as a viscous oily substance.

557 mg (1.45 mmol) of the above β -O-benzyl- α -L-aspartyl-L-phenylalanine methyl ester were dissolved in 15 ml of tetrahydrofuran (THF) to yield a solution which was maintained at 0 °C. To this solution were added 432 mg (1.45 mmol) of 3-(3-benzyloxy-4-methoxyphenyl)-3-methylbutyl aldehyde, 0.083 ml (1.45 mmol) of acetic acid and 462 mg (2.18 mmol) of NaB(OAc)₃H and stirred for one hour at 0 °C and overnight at room temperature'. To the reaction solution were added 50 ml of a saturated aqueous solution of sodium hydrogen carbonate and extraction was made twice with 50 ml of ethyl acetate. An organic layer was washed with a saturated saline water and dried over anhydrous magnesium sulfate. Magnesium sulfate was filtered off and the liquid filtrate was concentrated under reduced pressure. The residue was purified with preparative thin layer chromatography (PTLC) to yield 832 mg (1.25 mmol) of N-[N-[3-(3-benzyloxy-4-methoxyphenyl) -3-methylbutyl] - β -O-benzyl-L- α -aspartyl]-L-phenylalanine 1-methylester as a viscous oily substance.

The above 832 mg (1.25 mmol) of N-[N-[3-(3-benzyloxy-4-methoxyphenyl)-3-methylbutyl]- β -O-benzyl-L-α-aspartyl]-L-phenylalanine 1-methyl ester were dissolved in a mixed solvent of 25 ml of methanol and 2 ml of water, and 350 mg of 10% palladium carbon (containing 50% of water) were added thereto. The resulting mixture was reduced at room temperature for three hours under a hydrogen atmosphere. The catalyst was filtered off and the filtrate was concentrated under reduced pressure. The residue was purified with PTLC to remove an odor adsorbed to yield 400 mg (0.82 mmol) of N-[N-[3-(3-hydroxy-4-methoxyphenyl)-3-methylbutyl]-L- α-aspartyl]-L-phenylalanine 1-methylester as a solid substance.

¹HMMR (DMSO-d₆) δ :1.14 (s, 6H), 1.54-1.68 (m, 2H), 2.04-2.22 (m, 3H), 2.24-2.34 (dd, 1H), 2.84-2.94 (dd, 1H), 3.00-3.08 (dd, 1H), 3.31-3.36 (m, 1H), 3.59 (s, 3H), 3.71 (s, 3H), 4.46-4.55 (m, 1H), 6.60-6.65 (dd, 1H), 6.73 (s, 1H), 6.80 (d, 1H), 7.10-7.28 (m, 5H), 8.45 (d, 1H), 8.75 (brs, 1H)

ESI(Electrospray Ionization)-MS 487.3 (MH⁺)

Sweetness (sweetening potency), 70000 times the sweetness of sugar

### (Example 2)

### Synthesis of N- [N- [3- (4-methoxyphenyl) -3-methylbutyl] -L- α -aspartyl]-L-phenylalanine 1-methyl ester (Table 1, compound number 7)

N-[N-[3-(4-methoxyphenyl)-3-methylbutyl]-L-α-aspartyl]-L-phenylalanine 1-methyl ester was obtained as a solid substance, with a total yield of 72.2%, in the same way as in Example 1, except using 3-(4-methoxyphenyl)-3-methylbutyl aldehyde in place of 3-(3-benzyloxy-4-methoxyphenyl)-3-methylbutyl aldehyde.

¹HMMR (DMSO-d₆) δ :1.17 (s, 6H), 1.62-1.72 (m, 2H), 2.04-2.20 (m, 3H), 2.24- 2.34 (dd, 1H), 2.84-2.94 (dd, 1H), 2.95-3.07 (dd, 1H), 3.30-3.35 (m, 1H), 3.51 (s, 3H), 3.70 (s, 3H), 4.46-4.54 (m, 1H), 6.83 (d, 2H). 7.14-7.28 (m, 7H), 8.43 (d, 1H).
ESI-MS 471.3 (MH⁺)
Sweetness, 25000 times the sweetness of sugar

### (Example 3)

### Synthesis of N-[N-[3-(4-hydroxyphenyl)-3-methylbutyl] -L- α-aspartyl] -L-phenylalanine 1 methyl ester (Table 1, compound number 8)

N-[N-[3-(4-hydroxyphenyl) -3-methylbutyl] -L- α-aspartyl]-L-phenylalanine 1-methyl ester was obtained as a solid substance, with a total yield of 64.5%, in the same way as in Example 1, except using 3-(4-benzyloxyphenyl)-3-methylbutyl aldehyde in place of 3-(3-benzyloxy-4-methoxyphenyl)-3-methylbutyl aldehyde.
¹HMMR (DMSO-d₆) δ 1.15 (s, 6H), 1.58-1.72 (m, 2H), 2.04-2.20 (m, 3H), 2.24- 2.34 (dd, 1H), 2.85-2.94 (dd, 1H), 3.00-3.08 (dd, 1H), 3.30-3.36 (m, 1H), 3.59 (s, 3H), 4.46-4.55 (m, 1H), 6.67 (d, 2H), 7.07 (d, 2H), 7.10-7.27 (m, 5H), 8.44 (d, 1H), 9.15 (brs, 1H).
ESI-MS 457.3 (MH⁺)
Sweetness, 25000 times the sweetness of sugar

### (Example 4)

### Synthesis of N-[N-[3-(3-methoxy-4-hydroxyphenyl)-3-methylbutyl]-L-α-aspartyl]-L-phenylalanine 1 methyl ester (Table 1, compound number 9)

N-[N-[3-methoxy-4-hydroxyphenyl)-3-methylbutyl]-L-α-aspartyl]-L-phenylalanine 1-methyl ester was obtained as a solid substance, with a total yield of 62.2%, in the same way as in Example 1, except using 3-(3-methoxy-4-benzyloxyphenyl)-3-methylbutyl aldehyde in place of 3-(3-benzyloxy-4-methoxyphenyl)-3-methylbutyl aldehyde. ¹HMMR (DMSO-d₆) δ :1.17 (s, 6H), 1.63-1.72 (m, 2H), 2.08-2.22 (m, 3H), 2.25-2.33 (dd, 1H), 2.86-2.94 (dd, 1H), 3.00-3.08 (dd, 1H), 3.33-3.38 (m, 1H), 3.59 (s, 3H), 3.75 (s. 3H), 3.47-3.55 (m, 1H), 6.67 (s, 2H), 6.81 (s, 1H), 7.14-7.27 (m, 5H), 8.46 (d, 1H), 8.70 (brs, 1H).
ESI-MS 487.3 (MH⁺)
Sweetness, 40000 times the sweetness of sugar

### (Example 5)

### Synthesis of N-[N-[3-(3-hydroxy-4-methoxyphenyl)-3-methylbutyl]-L- α-aspartyl]-L-(α-methyl) phenylalanine 1-methylester (Table 1, compound number 22)

N-[N-[3-(3-hydroxy-4-methoxyphenyl)-3-methylbutyl]-L-α-aspartyl]-L-(α-methyl) phenylalanine 1-methyl ester was obtained as a solid substance, with a total yield of 77.2%, in the same way as in Example 1, except using N-t-butoxycarbonyl-β -O-benzyl- α -L-aspartyl-L-( α -methyl) phenylalanine methyl ester in place of N-t-butoxy carbonyl-β-O-benzyl-α-L-aspartyl-L-phenylalanine methyl ester. ¹HMMR (DMSO-d₆) δ :1.18 (s, 6H), 1.22 (s, 3H), 1.66-1.76 (m, 2H), 2.18-2.38 (m, 4H), 3.00 (d, 1H), 3.19 (d, 1H), 3.36-3.42 (m, 1H), 3.49 (s, 3H), 3.72 (s, 3H), 6.67 (dd, 1H). 6.74 (d, 1H), 6.80 (d, 1H), 7.02-7.06 (m, 2H), 7.20-7.30 (m, 3H), 8.29 (brs, 1H), 8.75 (brs, 1H).
ESI-MS 501.3 (MH⁺)
Sweetness, 40000 times the sweetness of sugar

### (Example 6 (comparative))

### Synthesis of N- [N- [3- (2-hydroxyphenyl) -3-methylbutyl]-L-α -aspartyl] -L-phenylalanine 1-methyl ester (Table 1, compound number 13)

N- [N- [3-(2-hydroxyphenyl)-3-methylbutyi]-L- α-aspartyl]-L-phenylalanine 1-methyl ester was obtained as a solid substance, with a total yield of 64.5%, in the same way as in Example 1, except using 3-(2-benzyloxyphenyl)-3-methylbutyl aldehyde in place of 3 - (3-benzyloxy - 4 - methoxyphenyl)-3-methylbutyl aldehyde
¹HMMR (DMSO-d₆) δ 1.26 (s, 6H), 1.84-2.30 (m, 6H), 2.88 (dd, 1H), 3.02 (dd, 1H), 3.32-3.38 (m, 1H), 3.59 (s, 3H), 4.45-4.54 (m, 1H), 6.68-6.78 (m, 3H), 6.96-7.06 (m, 2H), 7.12-7.30 (m, 5H), 8.50 (d, 1H), 9.30 (brs, 1H).
ESI-MS 457.4 (MH⁺)
Sweetness, 8000 times the sweetness of sugar

### (Example 7)

### Synthesis of N-[N-[3-(2-hydroxy-4-methoxyphenyl)-3-methylbutyl]-L-α-aspartyl]-L-phenylalanine 1- methyl ester (Table 1, compound number 14)

N-[N-[3-(2-hydroxy-4-methoxyphenyl)-3-methylbutyl]-L-α-aspartyl]-L-phenylalanine 1 methyl ester was obtained as a solid substance, with a total yield of 44.1%, in the same way as in Example 1, except using 3-(2-benzyloxy-4-methoxyphenyl)-3-methylbutyl aldehyde in place of 3-(3-benzyloxy-4-methoxy phenyl)-3-methylbutyl aldehyde.
¹HMMR (DMSO-d₆) δ :1.22 (s, 6H), 1.82-2.20 (m, 5H), 2.26 (dd, 1H), 2.88 (dd, 1H), 3.01 (dd, 1H), 3.34-3.40 (m, 1H), 3.59 (s, 3H), 3.64 (s, 3H), 4.46-4.53 (m, 1H), 6.28 (dd, 1H), 6.36 (d, 1H), 6.92 (d, 1H), 7.14-7.26 (m, 5H), 8.52 (d, 1H), 9.40 (brs, 1H).
ESI-MS 487.3 (MH⁺)
Sweetness, 20000 times the sweetness of sugar

### (Example 8)

### Synthesis of N-[N-[3-(2-hydroxy-4-methylphenyl)-3-methylbutyl]-L-α-aspartyl]-L-phenylalanine 1-methyl ester (Table 1, compound number 15)

N-[N-[3-(2-hydroxy-4-methylphenyl)-3-methylbutyl]-L-α-aspartyl]-L-phenylalanine 1-methyl ester was obtained as a solid substance, with a total yield of 45.1%, in the same way as in Example 1, except using 3-(2-benzyloxy-4-methylphenyl)-3-methylbutyl aldehyde in place of 3-(3-benzyloxy-4-methoxyphenyl)-3-methylbutyl aldehyde.
¹HMMR (DMSO-d₆) δ :1.23 (s, 6H), 1.82-2.20 (m, 5H), 2.14 (s, 3H), 2.25 (dd, 1H), 2.88 (dd, 1H), 3.01 (dd, 1H), 3.33-3.39 (m, 1H) , 3.58 (s, 3H), 4.46-4.54 (m, 1H), 6.51 (d, 1H), 6.87 (s, 1H), 6.90 (d, 1H), 7.10-7.23 (m, 5H), 8.51 (d, 1H), 9.20 (brs, 1H).
ESI-MS 471.2 (MH⁺)
Sweetness, 25000 times the sweetness of sugar

### (Example 9)

### Synthesis of N-[N-[3-(3,4-methylenedioxyphenyl)-3-methylbutyl]-L-α-aspartyl]-L-phenylalanine 1- methyl ester (Table 1, compound number 16)

N-[N-[3-(3,4-methylenedioxyphenyl)-3-methylbutyl]-L-α-aspartyl]-L-phenylalanine 1-methyl ester was obtained as a solid substance, with a total yield of 69.7%, in the same way as in Example 1, except using 3-(3,4-methylenedioxy phenyl-3-methylbutyl aldehyde in place of 3-(3-benzyloxy-4 -methoxyphenyl)-3-methylbutyl aldehyde.
¹HMMR (DMSO-d₆) δ :1.16 (s, 6H), 1.60-1.70 (m, 2H), 2.05-2.20 (m, 3H), 2.27 (dd, 1H), 2.89 (dd, 1H), 3.03 (dd, 1H), 3.31-3.35 (m, 1H), 3.59 (s, 3H), 4.46-4.54 (m, 1H), 5.94 (s, 2H), 6.72 (dd, 1H), 6.79 (d, 1H), 6.88 (d, 1H), 7.15-7.28 (m, 5H), 8.44 (d, 1H).
ESI-MS 485.4 (MH⁺)
Sweetness, 30000 times the sweetness of sugar

### (Example 10)

### Synthesis of N-[N-[3-(3-methyl-4-methoxyphenyl)-3-methylbutyl] -L-α-aspartyl]-L-phenylalanine 1- methyl ester (Table 1, compound number 17)

N- [N- [3- (3- methyl-4-methoxyphenyl)-3-methylbutyl]-L-α-aspartyl]-L-phenylalanine 1-methyl ester was obtained as a solid substance, with a total yield of 66.0%, in the same way as in Example 1, except using 3-(3-methyl-4-methoxyphenyl)-3-methylbutyl aldehyde in place of 3-(3-benzyloxy-4-methoxy phenyl)-3-methylbutyl aldehyde.
¹HMMR (DMSO-d₆) δ :1.16 (s, 6H), 1.63-1.72 (m, 2H), 2.13 (s, 3H), 2.08-2.20 (m, 3H), 2.25-2.32 (dd, 1H), 2.85-2.95 (dd, 1H), 3.00-3.06 (dd, 1H), 3.31-3.36 (m, 1H), 3.59 (s. 3H), 3.73 (s, 3H), 4.47-4.55 (m, 1H), 6.79-6.82 (m, 1H), 7.03-7.06 (m, 2H), 7.15-7.27 (m, 5H), 8.44-8.47 (d, 1H).
ESI-MS 485.5 (MH⁺)
Sweetness, 30000 times the sweetness of sugar

### (Example 11)

### Synthesis of N-[N-[3-(3-methyl-4-hydroxyphenyl)-3-methylbutyl]-L-α-aspartyl]-L-phenylalanine 1-methyl ester (Table 1, compound number 18)

N -[N-[3-(3-methyl-4-hydroxyphenyl)-3-methylbutyl] -L- α-aspartyl]-L-phenylalanine 1-methyl ester was obtained as a solid substance, with a total yield of 63.2%, in the same way as in Example 1, except using 3-(3-methyl-4-benzyloxyphenyl)-3-methylbutyl aldehyde in place of 3-(3-benzyloxy-4-methoxyphenyl)-3-methylbutyl aldehyde.
¹HMMR (DMSO-d₆) δ :1.14 (s, 6H), 1.59-1.68 (m, 2H), 2.09 (s, 3H), 2.09-2.18 (m, 3H), 2.25 (dd, 1H), 2.90 (dd, 1H), 3.02 (dd, 1H), 3.30-3.36 (m, 1H), 3.59 (s, 3H), 4.46-4.54 (m, 1H), 6.68 (d, 1H), 6.88 (dd, 1H), 6.96 (s, 1H), 6.14-6.73 (m, 5H), 8.46 (d, 1H), 9.01 (brs, 1H).
ESI-MS 471.4 (MH⁺)
Sweetness, 70000 times the sweetness of sugar

### (Example 12)

### Synthesis of N- [N- [2- [1- (3-methoxy-4-hydroxyphenyl) cyclopentyl] ethyl]-L- α -aspartyl]-L-phenylalanine 1-methyl ester (Table 1, compound number 20)

N-[N-[2-[1-(3-methoxy-4-hydroxyphenyl) cyclopentyl] ethyl]-L-α-aspartyl]-L-phenylalanine 1-methyl ester was obtained as a solid substance, with a total yield of 68.4%, in the same way as in Example 1, except using 2- [1- (3-methoxy-4-hydroxyphenyl) cyclopentyl] acetaldehyde in place of 3-(3-benzyloxy-4-methoxyphenyl)-3-methylbutyl aldehyde. ¹HMMR (DMSO-d₆) δ:1.48-1.82 (m,10H), 2.00-2.16 (m, 3H), 2.24 (dd, 1H), 2.90 (dd, 1H), 3.01 (dd, 1H), 3.30-3.40 (m, 1H), 3.59 (s, 3H), 3.74 (s, 3H), 4.45-4.53 (m, 1H), 6.59 (dd, 1H), 6.65 (d, 1H), 6.75 (dd, 1H), 7.14-7.28 (m, 5H), 8.44 (d, 1H), 8.70 (brs, 1H).
ESI-MS 513.4 (MH⁺)
Sweetness, 30000 times the sweetness of sugar

### (Example 13)

### Synthesis of N-[N-[3-(3-hydroxy-4-methoxyphenyl)-3-methylbutyl]-L-α-aspartyl]-L-phenylalanine 1- ethyl ester (Table 1, compound number 21)

N-[N-[3-(3-hydroxy-4-methoxyphenyl)-3-methylbutyl] -L- α-aspartyl]-L-phenylalanine 1- ethyl ester was obtained as a solid substance, with a total yield of 56.1%, in the same way as in Example 1, except using N-t-butoxycarbonyl-β-O-benzyl-α-L-aspartyl-L-phenylalanine ethyl ester in place of N-t-butoxycarbonyl- β -O-benzyl- α -L-aspartyl-L-phenylalanine methyl ester.
¹HMMR (DMSO-d₆) δ :1.09 1.13 (m, 9H), 1.58-1.67 (m, 2H), 2.08-2.37 (m, 4H), 2.86-2.93 (dd, 1H), 2.99-3.06 (dd, 1H), 3.32-3.37 (m, 1H), 3.71 (s, 3H), 4.00-4.07 (m, 2H), 4.44- 4.51 (m, 1H), 6.62-6.65 (d, 1H), 6.74-6.81 (m, 2H), 7.15-7.27 (m, 5H), 8.46 (d, 1H), 8.78 (brs, 1H).
ESI-MS 501.3 (MH⁺)
Sweetness, 15000 times the sweetness of sugar

### (Example 14)

### Synthesis of N-[N-[(RS)-3-(3-methoxy-4-hydroxyphenyl) butyl]-L-α-aspartyl]-L-phenylalanine1-methyl ester (Table 1, compound number 3)

419 mg (1.09 mmol) of β -O-benzyl- α -L-aspartyl-L-phenylalanine methyl ester, obtained in the same way as in Example 1, were dissolved in 10 ml of THF and the resulting solution was maintained at 0 °C. To this solution were added 308 mg (1.09 mmol) of 3-(3-methoxy-4-benyloxyphenyl)-2-butenal, 0.062 ml (1.09 mmol) of acetic acid and 345 mg (1.63 mmol) of NaB(OAc)₃H and the resulting mixture was stirred at 0 °C for one hour and further stirred overnight at room temperature. To the reaction solution were added 30 ml of a saturated aqueous solution of sodium hydrogen carbonate and extraction was carried out twice with 30 ml of ethyl acetate. An organic layer was washed with saturated saline water and dried over anhydrous magnesium sulfate. After filtering magnesium sulfate off, the liquid filtrate was concentrated under reduced pressure. The residue was purified with preparative thin layer chromatography (PTLC) to obtain 534 mg (0.82 mmol) of N-[N-[3-(3-methoxy-4-benzyloxyphenyl)-2-butenyl]- β -O-benzyl-L-α -aspartyl]-L-phenylalanine 1-methyl ester as a viscous oily substance.

534 mg (0.82 mmol) of the above N-[N-[3-(3-methoxy-4-benzyloxyphenyl-2-butenyl)- β -O-benzyl-L- α-aspartyl]-L-phenylalanine 1-methyl ester were dissolved in a mixed solvent of 20 ml of methanol and 1 ml of water. To the resulting mixture were added 200 mg of 10% palladium carbon (containing 50% of water). The resulting mixture was reduced at room temperature for three hours in a hydrogen atmosphere. The catalyst was filtered off and the resulting filtrate was concentrated under reduced pressure. The residue was purified with PTLC to remove an odor adsorbed to obtain 269 mg ( 0.57 mmol ) of N-[N-[(RS)-3-(3-methoxy-4-hydroxyphenyl) butyl]-L- α -aspartyl]-L-phenylalanine 1-methyl ester as a solid substance.
¹HMMR (DMSO-d₆) δ :1.10 (2d, 3H), 1.50-1.60 (m, 2H), 2.10-2.40 (m, 4H), 2.55-2.65 (m, 1H), 2.85-2.95 (m, 1H), 3.03-3.09 (dd, 1H), 3.34-3.40 (m, 1H), 3.60 (s, 1.5H), 3.61 (s, 1.5H), 3.74 (s, 1.5H), 3.75 (s, 1.5H), 4.50-4.60 (m, 1H), 6.55 (d, 1H), 6.67 (d, 1H), 6.72 (s, 1H), 7.15-7.30 (m, 5H), 8.50 (brd, 1H), 8.70 (brs, 1H).
ESI-MS 473.3 (MH⁺)
Sweetness, 30000 times the sweetness of sugar

### (Example 15)

### Synthesis of N-[N-[(RS)-3-(4-methoxyphenyl) butyl]-L- α-aspartyl]-L-phenylalanine 1- methyl ester (Table 1, compound number 1)

N-[N-[(RS)-3-(4-methoxyphenyl) butyl]- L- α - aspartyl]-L-phenylalanine 1-methyl ester was obtained as a solid substance with a total yield of 37.3% in the same way as in Example 14 except using 3-(4-methoxyphenyl)-2-butenal in place of 3-(3-methoxy-4-benzyloxyphenyl)-2-butenal. ¹HMMR (DMSO-d₆) δ :1.09 (d, 1.5H), 1.11 (d, 1.5H), 1.54 (m, 2H), 2.17-2.23 (m, 3H), 2.28-2.38 (m, 1H), 2.64 (m, 1H), 2.85-2.95 (m, 1H), 3.02-3.10 (dd, 1H), 3.60 (s, 1.5H), 3.61 (s, 1.5H), 3.70 (s, 1H), 4.54 (m, 1H), 6.83 (d, 2H) , 7.07 (d, 2H), 7.18-7.28 (m, 5H).
ESI-MS 457.3 (MH⁺)
Sweetness, 16000 times the sweetness of sugar

### (Example 16 (comparative))

### Synthesis of N- [N- [(RS)-3-(3-hydroxyphenyl)butyl] -L- α-aspartyl]-L-phenylalanine 1 methyl ester (Table 1, compound number 2)

N- [N-[(RS)-3- (3-hydroxyphenyl) butyl]-L- α--aspartyl]-L-phenylalanine 1-methyl ester was obtained as a solid substance with a total yield of 31.1% in the same way as in Example 14 except using 3-(3-benzyloxyphenyl)-2-butenal in place of 3-(3-methoxy-4-benzyloxyphenyl)-2-butenal.
¹HMMR (DMSO-d₆) δ :1.09 (m, 3H), 1.55 (m, 2H), 2.10-2.24 (m, 3H), 2.26-2.34 (dd, 1H), 2.58 (m, 1H), 2.85-2.98 (m, 1H), 3.01-3.10 (dd, 1H), 3.60 (s, 1.5H), 3.61 (s, 1.5H), 4.53 (m, 1H), 6.55-6.62 (m, 3H), 7.05 (t, 1H), 7.16-7.30 (m, 5H), 8.47 (m, 1H), 8.75 (brs, 1H).
ESI-MS 443.2 (MH⁺)
Sweetness, 12000 times the sweetness of sugar

### (Example 17)

### Synthesis of N-[N-[(RS)-3-(3-hydroxy-4-methoxyphenyl) butyl]-L- α -aspartyl]-L-phenylalanine 1- methyl ester (Table 1, compound number 4)

N-[N-[(RS)-3-(3-hydroxy-4-methoxyphenyl) butyl]-L-α -aspartyl]-L-phenylalanine 1-methyl ester was obtained as a solid substance with a total yield of 38.8% in the same way as in Example 14 except using 3-(3-benzyloxy-4-methoxyphenyl)-2-butenal in place of 3-(3-methoxy-4-benzyloxyphenyl)-2-butenal.
¹HMMR (DMSO-d₆) δ :1.08 (m, 3H), 1.53 (m, 2H), 2.13-2.21 (m, 3H), 2.28 (dd, 1H), 2.56 (m, 1H), 2.86-3.00 (m, 1H), 3.02-3.12 (dd, 1H), 3.29-3.40 (m, 1H), 3.60 (s, 1.5H), 3.61 (s, 1.5H), 3.71 (s, 3H), 4.53 (m, 1H), 6.53 (d, 1H), 6.60 (d, 1H), 6.79 (d, 1H), 7.15-7.26 (m, 5H), 8.46 (m, 1H), 8.75 (brs, 1H).
ESI-MS 473.3 (MH⁺)
Sweetness, 50000 times the sweetness of sugar

### (Example 18)

### Synthesis of N-[N-[3-((RS)-3-hydroxy-4-methoxyphenyl) butyl]-L- α -aspartyl]-3-cyclohexyl-L-alanine 1-methyl ester (Table 1, compound number 6)

N-[N-[(RS)-3-(3-hydroxy-4-methoxyphenyl) butyl]-L-α -aspartyl]-3-cyclohexyl-L-alanine 1-methyl ester was obtained as a solid substance with a total yield of 41.7% in the same way as in Example 14 except using N-t-butoxycarbonyl- β -O-benzyl- α -L-aspartyl-3-cyclohexyl-L-alanine methyl ester in place of N-t-butoxycarbonyl-β-O-benzyl-α-L-aspartyl-L-phenylalanine methyl ester and also except using 3-(3-benzyloxy-4-methoxyphenyl)-2-butenal in place of 3-(3-methoxy-4-benzyloxyphenyl)-2-butenal.
¹HMMR (DMSO-d₆) δ : 0.75-1.34 (m, 5H), 1.11 (d, 3H), 1.50-1.70 (m, 10H), 2.18-2.28 (m, 2H), 2.35-2.45 (m, 2H), 2.58-2.65 (m, 1H) , 3.27-3.36 (m, 1H), 3.60 (m, 3H), 3.71 (s, 3H), 4.35 (m, 1H), 6.53-6.60 (m, 1H), 6.61 (d, 1H), 6.79 (d, 1H), 8.44 (m, 1H), 8.80 (brs, 1H).
ESI-MS 479.4 (MH⁺)
Sweetness, 40000 times the sweetness of sugar

### (Example 19)

### Synthesis of N-[N-[(RS)-3-(3-methoxy-4-hydroxyphenyl) butyl] -L- α -aspartyl]-L-tyrosine 1-methyl ester (Table 1, compound number 5)

N-[N-[(RS)-3-(3-methoxy-4-hydroxyphenyl)-butyl]-L-α -aspartyl]-L-tyrosine 1-methyl ester was obtained as a solid substance with a total yield of 37.5% in the same way as in Example 14 except using N-t-butoxycarbonyl-β-O-benzyl- a -L aspartyl-L-tyrosine methyl ester in place of N-t-butoxycarbonyl- β -O-benzyl- α -L-aspartyl-L-phenylalanine methyl ester.
¹HMMR (DMSO-d₆) δ :1.10 (d, 3H), 1.55 (m, 2H), 2.16-2.41 (m, 4H), 2.58 (m, 1H), 2.70-2.82 (m, 1H), 2.85-2.95 (dd, 1H), 3.58 (s, 3H), 3.78 (s, 3H), 4.43 (m, 1H), 6.53-6.75 (m, 5H), 6.96 (d, 2H), 8.49 (brd, 1H), 8.75 (brs, 1H), 9.80 (brs, 1H)
ESI-MS 489.3 (MH⁺)
Sweetness, 25000 times the sweetness of sugar

### (Example 20)

### Synthesis of N- [N- [(RS)-3- (3-methyl-4-hydroxyphenyl) butyl]-L-α -aspartyl]-L-phenylalanine 1-methyl ester (Table 1, compound number 11)

N-[N-[(RS)-3-(3-methyl-4-hydroxy phenyl) butyl]-L-α -aspartyl] -L-phenylalanine 1-methyl ester was obtained as a solid substance with a total yield of 19.7% in the same way as in Example 14 except using 3-(3-methyl-4-benzyloxyphenyl)-2-butenal in place of 3- (3-methoxy-4-benzyloxyphenyl)-2-butenal
¹HMMR (DMSO-d₆) δ :1.06-1.09 (m, 3H), 1.49-1.54 (m, 2H), 2.08 (m, 3H), 2.11-2.20 (m, 3H), 2.17-2.33 (m, 1H), 2.85- 2.95 (m, 2H), 3.05-3.09 (m, 1H), 3.33-3-37 (m, 1H), 3.61 (s, 3H), 4.50-4.55 (m, 1H), 6.65 (m, 1H), 6.76 (m, 1H), 6.84 (s, 1H), 7.16-7.28 (m, 5H), 8.47-8.50 (m, 1H), 9.02 (brs, 1H)
ESI-MS 457.2 (MH⁺).
Sweetness, 50000 times the sweetness of sugar

### (Example 21 (comparative))

### Synthesis of N- [N-[3-(3-hydroxy-4-methoxyphenyl)-(RS)-2-methylpropyl]-L- α -aspartyl]-L-phenylalanine 1-methyl ester (Table 1, compound number 12)

N-[N-[-(3-hydroxy-4-methoxyphenyl)- (RS)-2-methylpropyl]-L- α -aspartyl]-L-phenylalanine 1-methyl ester was obtained as a solid substance with a total yield of 45.6% in the same way as in Example 14 except using 3-(3-benzyloxy-4-methoxyphenyl)-2-methyl-2-propenal in place of 3-(3-methoxy-4-benzyloxyphenyl)-2-butenal.
¹HMMR (DMSO-d₆) δ : 0.68-0.85 (m, 3H), 1.65-1.82 (m, 1H), 2.08- 2.37 (m, 2H), 2.27-2.30 (d, 4H), 2.94-3.10 (m, 2H), 3.43-3.45 (m, 1H), 3.62 (s, 3H), 3.72 (s. 3H), 4.48-4.59 (m, 1H), 6.49-6.59 (m, 2H), 6.77-6.80 (m, 1H), 7.20-7.29 (m, 5H), 8.57-8.58 (m, 1H), 8.92 (brs, 1H).
ESI-MS 473.4 (MH⁺)
Sweetness, 5000 times the sweetness of sugar

### (Example 22)

### Synthesis of N-[N-[3-(3-hydroxy-4-methylphenyl)-3-mehylbutyl]-L- α-aspartyl]-L-phenylalanine 1-methyl ester (Table 1, compound number 19)

274 mg (0.97 mmol) of 3-[(3-benzyloxy-4-methyl) phenyl]-3-methylbutyl aldehyde, 353 mg (1.2 mmol) of aspartame and 100 mg of 10% palladium carbon ( containing 50% of water ) were added to 7 ml of methanol and stirred at room temperature for four hours in a hydrogen atmosphere. The catalyst was filtered off and the resulting filtrate was concentrated under reduced pressure. The residue was purified by preparative thin layer chromatography (PTLC) to produce 299 mg (0.64 mmol, 65.5%) of N-[N-[3-(3-hydroxy-4-methylphenyl)-3-mehylbutyl]-L- α -aspartyl]-L-phenylalanine 1-methyl ester as a solid substance.
¹HMMR (DMSO-d₆) δ : 1.14 (s, 6H), 1.58-1,70 (m, 2H), 2.05 (s, 3H), 2.07-2.42 (m, 4H), 2.89 (dd, 1H), 3.03 (dd, 1H), 3.30-3.40 (m, 1H), 3.59 (s, 3H), 4.46-4.54 (m, 1H), 6.60 (d, 1H), 6.73 (s, 1H), 6.94 (d, 1H), 7.15-7.30 (m, 5H), 8.46 (brs, 1H) 9.08 (brs, 1H).
ESI-MS 471.3 (MH⁺)
Sweetness, 60000 times the sweetness of sugar

### (Example 23)

### Synthesis of N-[N-[3-(3,4-dihydroxyphenyl)-3-mehylbutyl]-L- α-aspartyl]-L-phenylalanine 1-methyl ester (Table 1, compound number 23)

N- [N- [3- (3,4-dihydroxyphenyl)-3-methylbutyl]-L- α-aspartyl]-L-phenylalanine 1-methyl ester was obtained as a solid substance with a total yield of 76.5% in the same way as in Example 1 except using 3-(3,4-dibenzyloxyphenyl)-3-methylbutyl aldehide in place of 3-(3-benzyloxy-4-methoxyphenyl)-3-methlbutyl aldehide.
¹HMMR (DMSO-d₆) δ : 1.14 (s, 6H), 1.76-1.93 (m, 2H), 2.40-2.50 (m, 2H), 2.73-2.80 (m, 2H), 2.91 (dd, 1H), 3.06 (dd, 1H), 3.59 (s, 3H), 3.95-4.05 (m, 1H) 4.45-4.55 (m, 1H), 6.52 (d, 1H), 6.64-6.70 (m, 2H), 6.94 (d, 1H), 7.15-7.30 (m, 5H), 8.73 (brs, 1H), 8.80 (brs, 1H), 9.09 (brs, 1H).
ESI-MS 473.3 (MH⁺)
Sweetness, 50000 times the sweetness of sugar

### Effect of Invention

The novel N-alkylaspartyl dipeptide ester derivative according to the present invention is low in calories and exhibits a sweetening potency which is particularly superior, in comparison with conventional sweetening agents. In the present invention, a novel chemical substance which has superior properties as a sweetening agent can be provided. The novel derivative can be used not only for a sweetening agent but also for the affording of sweetness to foods or the like products, such as beverages (drinks) and foods, requiring sweet taste.

## Claims

1. An N-alkylaspartyl dipeptide ester derivative, including its salt form, represented by the following general formula (1): 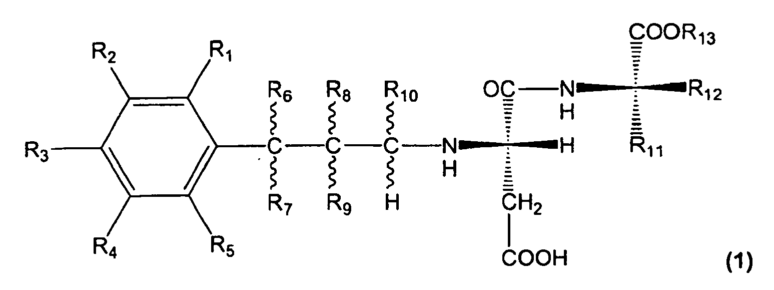 where R₁, R₂, R₄, and R₅ are reciprocally independent and denote a substituent selected from the group consisting of a hydrogen atom, a hydroxyl group, an alkoxy group having 1 to 3 carbon atoms, an alkyl group having 1 to 3 carbon atoms and a hydroxy alkyloxy group having two or three carbon atoms;
R₃ denotes a substituent selected from the group consisting of a hydroxyl group, an alkoxy group having 1 to 3 carbon atoms, an alkyl group having 1 to 3 carbon atoms and a hydroxy alkyloxy group having two or three carbon atoms;
or R₁ and R₂, or R₂ and R₃ combine together and denote a methylene dioxy group,
provided that in case that R₁ and R₂, or R₂ and R₃ combined together denote a methylene dioxy group, R₄ and R₅, and R₁ or R₃ which is not combined with R₂ together, are reciprocally independent and denote one of the above-mentioned substituents designated for the symbol;
R₆denotes an alkyl group with 1 to 3 carbon atoms; R₇, R₈, R₉ and R₁₀ are reciprocally independent and denote a substituent selected from the group consisting of a hydrogen atom and an alkyl group with 1 to 3 carbon atoms and optionally two substituents selected from R₆, R₇, R₈, R₉ and R₁₀ therein may combine together and denote an alkylene group with 1 to 5 carbon atoms;
provided that, if any optional two substituents selected from R₆, R₇, R₈, R₉ and R₁₀ combine together and denote an alkylene group with 1 to 5 carbon atoms, the substituents other than the selected two are reciprocally independent and denote one of said substituents designated for the symbol;
R₁₁ denotes a substituent selected from the group consisting of a hydrogen atom, a benzyl group, a p-hydroxy benzyl group, a cyclohexyl methyl group, a phenyl group, a cyclohexyl group, a phenyl ethyl group and a cyclohexyl ethyl group, and R₁₂ denotes a substituent selected from the group consisting of a hydrogen atom and an alkyl group with 1 to 3 carbon atoms, and R₁₃ denotes a substituent selected from the group consisting of alkyl groups with 1 to 4 carbon atoms

2. The derivative as defined in claim 1 wherein R₃ denotes a methoxy group, R₁, R₂, R₄, R₅, R₇, R₈, R₉, R₁₀ and R₁₂ denote a hydrogen atom, R₆ and R₁₃ denote a methyl group and R₁₁ denotes a benzyl group.

3. The derivative as defined in claim 1 wherein R₂ denotes a methoxy group, R₃ denotes a hydroxyl group, R₁, R₄, R₅ , R₇ , R₈ , R₉ , R₁₀ and R₁₂ denote a hydrogen atom, R₆ and R₁₃ denote a methyl group and R₁₁ denotes a benzyl group.

4. The derivative as defined in claim 1 wherein R₂ denotes a hydroxyl group, R₃ denotes a methoxy group, R₁, R₄, R₅, R₇, R₈ , R₉, R₁₀ and R₁₂ denote a hydrogen atom, R₆ and R₁₃ denote a methyl group and R₁₁ denotes a benzyl group.

5. The derivative as defined in claim 1 wherein R₂ denotes a methoxyl group, R₃ denotes a hydroxy group, R₁, R₄, R₅, R₇, R₈, R₉, R₁₀ and R₁₂ denote a hydrogen atom, R₆ and R₁₃ denote a methyl group and R₁₁ denotes a p-hydroxy benzyl group.

6. The derivative as defined in claim 1 wherein R₂ denotes a hydroxyl group, R₃ denotes a methoxy group, R₁, R₄, R₅, R₇, R₈, R₉, R₁₀ and R₁₂ denote a hydrogen atom, R₆ and R₁₃ denote a methyl group and R₁₁ denotes a cyclohexyl methyl group.

7. The derivative as defined in claim 1 wherein R₃ denotes a methoxy group, R₁, R₂, R₄, R₅, R₈, R₉, R₁₀ and R₁₂ denote a hydrogen atom, R₆, R₇ and R₁₃ denote a methyl group and R₁₁ denotes a benzyl group.

8. The derivative as defined in claim 1 wherein R₃ denotes a hydroxyl group, R₁, R₂, R₄, R₅, R₈, R₉, R₁₀ and R₁₂ denote a hydrogen atom, R₆, R₇ and R₁₃ denote a methyl group, and R₁₁ denotes a benzyl group.

9. The derivative as defined in claim 1 wherein R₂ denotes a methoxy group, R₃ denotes a hydroxyl group, R₁, R₄, R₅, R₈, R₉, R₁₀ and R₁₂ denote a hydrogen atom, R₆, R₇ and R₁₃ denote a methyl group, and R₁₁ denotes a benzyl group.

10. The derivative as defined in claim 1 wherein R₂ denotes a hydroxyl group, R₃ denotes a methoxy group, R₁, R₄, R₅, R₈, R₉, R₁₀ and R₁₂ denote a hydrogen atom, R₆, R₇ and R₁₃ denote a methyl group, and R₁₁ denotes a benzyl group.

11. The derivative as defined in claim 1 wherein R₂ denotes a methyl group, R₃ denotes a hydroxyl group, R₁, R₄, R₅, R₇, R₈, R₉, R₁₀ and R₁₂ denote a hydrogen atom, R₆ and R₁₃ denote a methyl group, and R₁₁ denotes a benzyl group.

12. The derivative as defined in claim 1 wherein R₁ denotes a hydroxyl group, R₃ denotes a methoxy group, R₂, R₄, R₅, R₈, R₉, R₁₀ and R₁₂ denote a hydrogen atom, R₆, R₇ and R₁₃ denote a methyl group, and R₁₁ denotes a benzyl group.

13. The derivative as defined in claim 1 wherein R₁ denotes a hydroxyl group, R₃ denotes a methyl group, R₂, R₄, R_{5'} R₈, R₉, R₁₀ and R₁₂ denote a hydrogen atom, R₆, R₇ and R₁₃ denote a methyl group, and R₁₁ denotes a benzyl group.

14. The derivative as defined in claim 1 wherein R₂ and R₃ combine together and denote a methylene dioxy group, R₁, R₄, R₅, R₈, R₉, R₁₀ and R₁₂ denote a hydrogen atom, R₆, R₇ and R₁₃ denote a methyl group, and R₁₁ denotes a benzyl group.

15. The derivative as defined in claim 1 wherein R₂ denotes a methyl group, R₃ denotes a methoxy group, R₁, R₄, R₅, R₈, R₉, R₁₀ and R₁₂ denote a hydrogen atom, R₆, R₇ and R₁₃ denote a methyl group, and R₁₁ denotes a benzyl group.

16. The derivative as defined in claim 1 wherein R₂ denotes a methyl group, R₃ denotes a hydroxyl group, R₁, R₄, R₅, R₈, R₉, R₁₀ and R₁₂ denote a hydrogen atom, R₆, R₇ and R₁₃ denote a methyl group, and R₁₁ denotes a benzyl group.

17. The derivative as defined in claim 1 wherein R₂ denotes a hydroxyl group, R₃ denotes a methyl group, R₁, R₄, R₅, R₈, R₉, R₁₀ and R₁₂ denote a hydrogen atom, R₆, R₇ and R₁₃ denote a methyl group, and R₁₁ denotes a benzyl group.

18. The derivative as defined in claim 1 wherein R₂ denotes a methoxy group, R₃ denotes a hydroxyl group, R₁, R₄, R₅, R₈, R₉, R₁₀ and R₁₂ denote a hydrogen atom, R₆ and R₇combine together and denote a tetramethylene group, R₁₁ denotes a benzyl group, and R₁₃ denotes a methyl group.

19. The derivative as defined in claim 1 wherein R₂ denotes a hydroxyl group, R₃ denotes a methoxy group, R₁, R₄, R₅, R₈, R₉, R₁₀ and R₁₂ denote a hydrogen atom, R₆ and R₇ denote a methyl group, R₁₁ denotes a benzyl group, and R₁₃ denotes an ethyl group

20. The derivative as defined in claim 1 wherein R₂ denotes a hydroxyl group, R₃ denotes a methoxy group, R₁, R₄, R₅, R₈, R₉ and R₁₀ denote a hydrogen atom, R₆, R₇, R₁₂ and R₁₃ denote a methyl group, and R₁₁ denotes a benzyl group.

21. The derivative as defined in claim 1 wherein R₂ and R₃ denote a hydroxyl group, R₁, R₄, R₅, R₈, R₉, R₁₀ and R₁₂ denote a hydrogen atom, R₆, R₇ and R₁₃ denote a methyl group, and R₁₁ denotes a benzyl group.

22. The derivative as defined in anyone claim of claims 1 to 6 and 11 wherein, if the substituents R₆ and R₇ differ from each other, the configuration of the carbon atom to which R₆ is linked in said formula is in the state of (R), (S) or (RS).

23. The derivative as defined in claim 1 wherein, if the substituents R₈ and R₉ differ from each other, the configuration of the carbon atom to which R₈ is linked in said formula is in the state of (R), (S) or (RS).

24. The derivative as defined in claim 1 wherein, if R₁₀ denotes a substituent other than a hydrogen atom, the configuration of the carbon atom to which R₁₀ is linked in said formula is in the state of (R), (S) or (RS).

25. A sweetening agent, or a sweetened food or the like product comprising the derivative as defined in claim 1 as an effective component, which may optionally contain a carrier or a bulking agent for the sweetening agents

26. A method for imparting sweetness to a product in need of sweetness, comprising the step of adding a derivative as claimed in claim 1 to said product.

27. The method of claim 26, wherein the product in need of sweetness is a food, confectionary, chewing gum, beverage, pharmaceutical, hygiene product, toiletry, cosmetic, or veterinary product for animals other than humans.

28. A method for manufacturing a compound of formula (1) as claimed in claim 1, where R₁₀ denotes a hydrogen atom, said method comprising a step of reacting an aldehyde shown by the general formula (2): 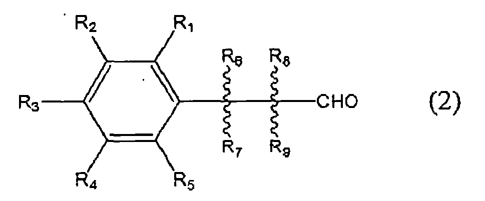 where R₁, R₂, R₃, R₄, R₅, R₆, R₇, R₈ and R₉ are as defined in formula (1) of claim 1;
with an aspartame derivative as shown by the following general formula (3); 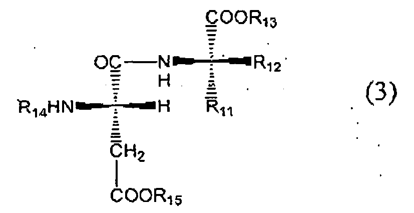 under a reductive alkylation condition;
wherein R₁₁, R₁₂ and, R₁₃ in formula (3) are as defined in formula (1) of claim 1;
R₁₄ denotes a hydrogen atom or substituent that can be converted into a hydrogen atom under the reductive alkylation condition; and
R₁₅ denotes a hydrogen atom, a benzyl group or a substituent that may be used for protecting a carboxyl group.

29. A method for manufacturing a compound of formula (1) as claimed in claim 1, where R₇, R₉ and R₁₀ denote a hydrogen atom, said method comprising a step of reacting an aldehyde shown by the general formula (4): 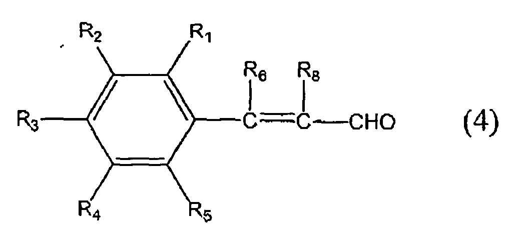 where R₁, R₂, R₃, R₄, R₅, R₆ and R₈ are as defined in formula (1) of claim 1;
with an aspartame derivative as shown by the following general formula (3): 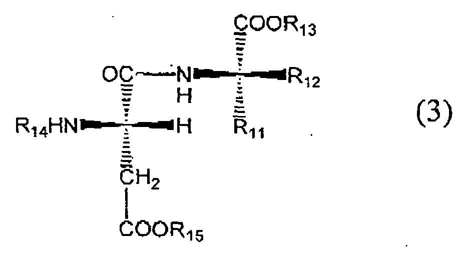 under a reductive alkylation condition;
wherein R₁₁, R₁₂ and R₁₃ in formula (3) are as defined in formula (1) of claim 1;
R₁₄ denotes a hydrogen atom or substituent that can be converted into a hydrogen atom under the reductive alkylation condition; and
R₁₅ denotes a hydrogen atom, a benzyl group or a substituent that may be used for protecting a carboxyl group.

30. A method for manufacturing a compound of formula (1) as claimed in claim 1, comprising a step of reacting a compound shown by the general formula (5): 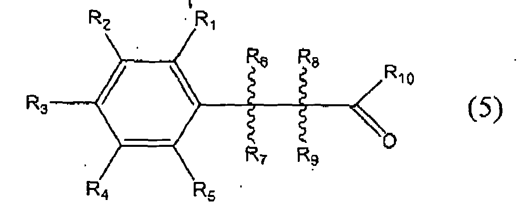 where R₁, R₂, R₃, R₄, R₅, R₆, R₈, R₉ and R₁₀ are as defined in formula (1) of claim 1;
with an aspartame derivative as shown by the following general formula (3): 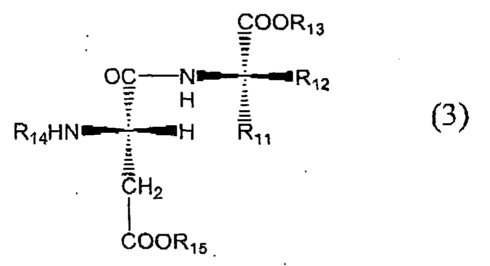 under a reductive alkylation condition;
wherein R₁₁, R₁₂ and R₁₃ in formula (3) are as defined in formula (1) of claim 1;
R₁₄ denotes a hydrogen atom or substituent that can be converted into a hydrogen atom under the reductive alkylation condition; and
R₁₅ denotes a hydrogen atom, a benzyl, group or a substituent that may be used for protecting a carboxyl group.

## Patentansprüche

1. N-Alkylaspartyldipeptid-Esterderivat, einschließlich dessen Salz, der folgenden allgemeinen Formel (1) 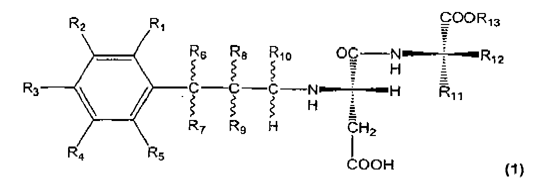 worin R₁, R₂, R₄ und R₅ unabhängig voneinander sind und einen Substituenten darstellen, der aus der Gruppe ausgewählt ist, die aus einem Wasserstoffatom, einer Hydroxygruppe, einer Alkoxygruppe mit 1 bis 3 Kohlenstoffatomen, einer Alkylgruppe mit 1 bis 3 Kohlenstoffatomen und einer Hydroxyalkyloxygruppe mit 2 oder 3 Kohlenstoffatomen besteht;
R₃ einen Substituenten darstellt, der aus der Gruppe ausgewählt ist, die aus einer Hydroxygruppe, einer Alkoxygruppe mit 1 bis 3 Kohlenstoffatomen, einer Alkylgruppe mit 1 bis 3 Kohlenstoffatomen und einer Hydroxyalkyloxygruppe mit 2 oder 3 Kohlenstoffatomen besteht;
oder R₁ und R₂ oder R₂ und R₃ miteinander verbunden sind und eine Methylendioxygruppe darstellen,
mit der Maßgabe, dass in dem Fall, dass die miteinander verbundenen R₁ und R₂ oder R₂ und R₃ eine Methylendioxygruppe darstellen, R₄ und R₅ und R₁ oder R₃, welches nicht an R₂ gebunden ist, unabhängig voneinander sind und einen der vorstehend erwähnten jeweiligen Substituenten darstellen; R₆ eine Alkylgruppe mit 1 bis 3 Kohlenstoffatomen darstellt; R₇, R₈, R₉ und R₁₀ unabhängig voneinander sind und einen Substituenten darstellen, der aus der Gruppe ausgewählt ist, die aus einem Wasserstoffatom und einer Alkylgruppe mit 1 bis 3 Kohlenstoffatomen besteht, und optional können zwei Substituenten, die unter R₆, R₇, R₈, R₉ und R₁₀ ausgewählt sind, miteinander verbunden sein und eine Alkylengruppe mit 1 bis 5 Kohlenstoffatomen darstellen;
mit der Maßgabe, dass wenn beliebige optionale zwei Substituenten, die unter R₆, R₇ R₈, R₉ und R₁₀ ausgewählt sind, miteinander verbunden sind und eine Alkylengruppe mit 1 bis 5 Kohlenstoffatomen darstellen, die anderen Substituenten als die ausgewählten zwei jeweils unabhängig voneinander sind und einen der jeweils für das Symbol angegebenen Substituenten darstellen;
R₁₁ einen Substituenten darstellt, der aus der Gruppe ausgewählt ist, die aus einem Wasserstoffatom, einer Benzylgruppe, einer p-Hydroxybenzylgruppe, einer Cyclohexylmethylgruppe, einer Phenylgruppe, einer Cyclohexylgruppe, einer Phenylethylgruppe und einer Cyclohexylethylgruppe besteht, und R₁₂ einen Substituenten darstellt, der aus der Gruppe ausgewählt ist, die aus einem Wasserstoffatom und einer Alkylgruppe mit 1 bis 3 Kohlenstoffatomen besteht, und R₁₃ einen Substituenten darstellt, der aus der Gruppe ausgewählt ist, die aus Alkylgruppen mit 1 bis 4 Kohlenstoffatomen besteht.

2. Das Derivat nach Anspruch 1, worin R₁ eine Methoxygruppe darstellt, R₁, R₂, R₄, R₅, R₇, R₈, R₉, R₁₀ und R₁₂ ein Wasserstoffatom darstellen, R₆ und R₁₃ eine Methylgruppe darstellen und R₁₁ eine Benzylgruppe darstellt.

3. Derivat nach Anspruch 1, worin R₂ eine Methoxygruppe darstellt, R₃ eine Hydroxygruppe darstellt, R₁, R₄, R₅, R₇, R₈, R₉, R₁₀ und R₁₂ ein Wasserstoffatom darstellen, R₆ und R₁₃ eine Methylgruppe darstellen und R₁₂ eine Benzylgruppe darstellt.

4. Derivat nach Anspruch 1, worin R₂ eine Hydroxygruppe darstellt, R₃ eine Methoxygruppe darstellt, R₁, R₄, R₅, R₇, R₈, R₉, R₁₀ und R₁₂ ein Wasserstoffatom darstellen, R₆ und R₁₃ eine Methylgruppe darstellen und R₁₁ eine Benzylgruppe darstellt.

5. Derivat nach Anspruch 1, worin R₂ eine Methoxygruppe darstellt, R₃ eine Hydroxygruppe darstellt, R₁, R₄, R₅, R₇, R₈, R₉, R₁₀ und R₁₂ ein Wasserstoffatom darstellen, R₆ und R₁₃ eine Methylgruppe darstellen und R₁₁ eine p-Hydroxybenzylgruppe darstellt.

6. Derivat nach Anspruch 1, worin R₂ eine Hydroxygruppe darstellt, R₃ eine Methoxygruppe darstellt, R₁, R₄, R₅, R₇, R₈, R₉, R₁₀ und R₁₂ ein Wasserstoffatom darstellen, R₆ und R₁₃ eine Methylgruppe darstellen und R₁₁ eine Cyclohexylmethylgruppe darstellt.

7. Derivat nach Anspruch 1, worin R₃ eine Methoxygruppe darstellt, R₁, R₂, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

8. Derivat nach Anspruch 1, worin R₃ eine Hydroxygruppe darstellt, R₁, R₂, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

9. Derivat nach Anspruch 1, worin R₂ eine Methoxygruppe darstellt, R₃ eine Hydroxygruppe darstellt, R₁, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

10. Derivat nach Anspruch 1, worin R₂ eine Hydroxygruppe darstellt, R₃ eine Methoxygruppe darstellt, R₁, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

11. Derivat nach Anspruch 1, worin R₂ eine Methylgruppe darstellt, R₃ eine Hydroxygruppe darstellt, R₁, R₄, R₅, R₇, R₈, R₉, R₁₀ und R₁₂ ein Wasserstoffatom darstellen, R₆ und R₁₃ eine Methylgruppe darstellen und R₁₁ eine Benzylgruppe darstellt.

12. Derivat nach Anspruch 1, worin R₁ eine Hydroxygruppe darstellt, R₃ eine Methoxygruppe darstellt, R₂, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

13. Derivat nach Anspruch 1, worin R₁ eine Hydroxygruppe darstellt, R₃ eine Methylgruppe darstellt, R₂, R₉, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

14. Derivat nach Anspruch 1, worin R₂ und R₃ miteinander verbunden sind und eine Methylendioxygruppe darstellen, R₁, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

15. Derivat nach Anspruch 1, worin R₂ eine Methylgruppe darstellt, R₃ eine Methoxygruppe darstellt, R₁, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

16. Derivat nach Anspruch 1, worin R₂ eine Methylgruppe darstellt, R₃ eine Hydroxygruppe darstellt, R₁, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

17. Derivat nach Anspruch 1, worin R₂ eine Hydroxygruppe darstellt, R₃ eine Methylgruppe darstellt, R₁, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

18. Derivat nach Anspruch 1, worin R₂ eine Methoxygruppe darstellt, R₃ eine Hydroxygruppe darstellt, R₁, R₄, R₅, R₈, R₉, R₁₀ und R₁₂ ein Wasserstoffatom darstellen, R₆ und R₇ miteinander verbunden sind und eine Tetramethylengruppe darstellen, R₁₁ eine Benzylgruppe darstellt und R₁₃ eine Methylgruppe darstellt.

19. Derivat nach Anspruch 1, worin R₂ eine Hydroxygruppe darstellt, R₃ eine Methoxygruppe darstellt, R₁, R₄, R₅, R₈, R₉, R₁₀ und R₁₂ ein Wasserstoffatom darstellen, R₆ und R₇ eine Methylgruppe darstellen, R₁₁ eine Benzylgruppe darstellt und R₁₃ eine Ethylgruppe darstellt.

20. Derivat nach Anspruch 1, worin R₂ eine Hydroxygruppe darstellt, R₃ eine Methoxygruppe darstellt, R₁, R₄, R₅, R₈, R₉ und R₁₀ ein Wasserstoffatom darstellen, R₆, R₇, R₁₂ und R₁₃ eine Methylgruppe darstellen und R₁₁ eine Benzylgruppe darstellt.

21. Derivat nach Anspruch 1, worin R₂ und R₃ eine Hydroxygruppe darstellen, R₁, R₄, R₅, R₈, R₉, R₁₀ und R₁₂ ein Wasserstoffatom darstellen, R₆, R₇ und R₁₃ eine Methylgruppe darstellen und R₁₁ eine Benzylgruppe darstellt.

22. Derivat nach einem der Ansprüche 1 bis 6 und 11, worin, wenn die Substituenten R₆ und R₇ sich voneinander unterscheiden, die Konfiguration des Kohlenstoffatoms, an den R₆ gebunden ist, in der Formel im Zustand (R), (S) oder (RS) ist.

23. Derivat nach Anspruch 1, worin, wenn die Substituenten R₈ und R₉ sich voneinander unterscheiden, die Konfiguration des Kohlenstoffatoms, an das R₈ gebunden ist, in der Formel im Zustand von (R), (S) oder (RS) ist.

24. Derivat nach Anspruch 1, worin, wenn R₁₀ einen anderen Substituenten als ein Wasserstoffatom darstellt, die Konfiguration des Kohlenstoffatoms, an das R₁₀ gebunden ist, in der Formel im Zustand von (R), (S) oder (RS) ist.

25. Süßungsmittel oder gesüßtes Nahrungsmittel oder ein ähnliches Produkt, welches das Derivat nach Anspruch 1 als wirksame Komponente enthält, welches gegebenenfalls einen Träger oder einen Füllstoff für die Süßungsmittel enthalten kann.

26. Verfahren zum Übertragen von Süße auf ein Produkt, welches einer Süße bedarf, wobei das Verfahren die Stufe umfasst, bei der ein Derivat nach Anspruch 1 zu dem Produkt gegeben wird.

27. Verfahren nach Anspruch 26, wobei das Produkt, das einer Süße bedarf, ein Nahrungsmittel, Süßwaren, Kaugummi, Getränke, Arzneimittel, Hygieneprodukte, Toilettenartikel, Kosmetika oder tiermedizinische Produkte für Tiere sind.

28. Verfahren zur Herstellung einer Verbindung der Formel (1) nach Anspruch 1, wobei R₁₀ ein Wasserstoff darstellt, wobei das Verfahren eine Stufe umfasst, bei der ein Aldehyd der allgemeinen Formel (2): 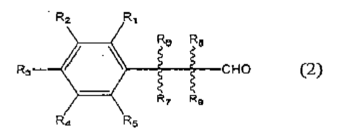 worin R₁, R₂, R₃, R₄, R₅, R₆, R₇, R₈ und R₉ wie in Formel (1) des Anspruchs 1 definiert sind;
mit einem Aspartamderivat der folgenden allgemeinen Formel (3) : 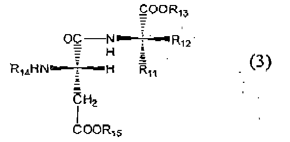 unter einer reduktiven Alkylierungsbedingung umgesetzt wird;
worin R₁₁, R₁₂ und R₁₃ in Formel (3) wie in Formel (1) des Anspruchs 1 definiert sind;
R₁₄ ein Wasserstoffatom oder einen Substituenten darstellt, der unter der reduktiven Alkylierungsbehandlung in ein Wasserstoffatom umgewandelt werden kann; und
R₁₅ ein Wasserstoffatom, eine Benzylgruppe oder einen zum Schützen einer Carboxygruppe einsetzbaren Substituenten darstellt.

29. Verfahren zum Herstellen einer Verbindung der Formel (1) nach Anspruch 1, wobei R₇, R₉ und R₁₀ ein Wasserstoffatom darstellen, wobei das Verfahren eine Stufe umfasst, bei der ein Aldehyd der allgemeinen Formel (4) 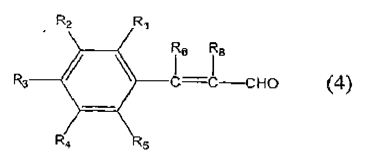 worin R₁, R₂, R₃, R₄, R₅, R₆, und R₈ wie in Formel (1) des Anspruchs 1 definiert sind;
mit einem Aspartamderivat der folgenden allgemeinen Formel (3) : 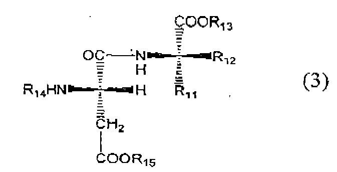 unter einer reduktiven Alkylierungsbehandlung umgesetzt wird; worin R₁₁, R₁₂ und R₁₃ in Formel (3) wie in Formel (1) des Anspruchs 1 definiert sind;
R₁₄ ein Wasserstoffatom oder einen Substituenten darstellt, der unter der reduktiven Alkylierungsbehandlung in ein Wasserstoffatom umgewandelt werden kann; und
R₁₅ ein Wasserstoffatom, eine Benzylgruppe oder einen zum Schützen einer Carboxygruppe einsetzbaren Substituenten darstellt.

30. Verfahren zum Herstellen einer Verbindung der Formel (1) nach Anspruch 1, welches die Stufe umfasst, bei der eine Verbindung der allgemeinen Formel (5) 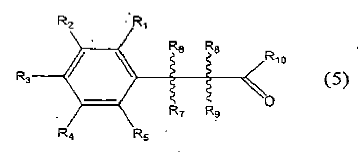 worin R₁, R₂, R₃, P₄, R₅, R₆, R₉, R₉ und R₁₀ die in Formel (1) des Patentanspruchs 1 definiert sind;
mit einem Asparatamderivat der folgenden allgemeinen Formel (3) : 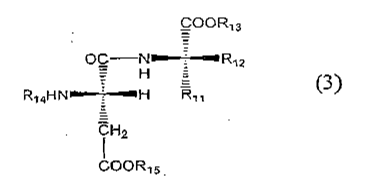 unter einem reduktiven Alkylierungszustand umgesetzt wird;
worin R₁₁, R₁₂ und R₁₃ in Formel (3) wie in Formel (1) des Patentanspruchs 1 definiert sind;
R₁₄ ein Wasserstoffatom oder einen Substituenten darstellt, der unter der reduktiven Alkylierungsbehandlung in ein Wasserstoffatom umgewandelt werden kann; und
R₁₅ ein Wasserstoffatom, eine Benzylgruppe oder einen zum Schützen einer Carboxygruppe einsetzbaren Substituenten darstellt.

## Revendications

1. Dérivé d'ester de N-alkylaspartyldipeptlde, comprenant sa forme de sel, représenté par la formule générale (1) suivante : 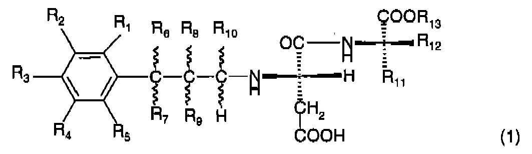 dans laquelle R₁, R₂, R₄ et R₅ sont réciproquement Indépendants et représentent un substituant choisi parmi un atome d'hydrogène, un groupe hydroxyle, un groupe alcoxy ayant de 1 à 3 atomes de carbone, un groupe alkyle ayant de 1 à 3 atomes de carbone et un groupe hydroxyalkyloxy ayant 2 ou 3 atomes de carbone ;
R₃ représente un substituant choisi parmi un groupe hydroxyle, un groupe alcoxy ayant de 1 à 3 atomes de carbone, un groupe alkyle ayant de 1 à 3 atomes de carbone et un groupe hydroxyalkyloxy ayant 2 ou 3 atomes de carbone ;
ou R₁ et R₂, ou R₂ et R₃ sont combinés ensemble et représentent un groupe méthylène dioxy,
à condition que dans le cas où R₁ et R₂, ou R₂ et R₃ combinés ensemble représentent un groupe méthylènedloxy, R₄ et R₅, et R₁ ou R₃ qui ne sont pas combinés ensemble avec R₂, sont réciproquement Indépendants et représentent un des substltuants cités ci-dessus désignés pour le symbole ;
R₆ représente un groupe alkyle avec de 1 à 3 atomes de carbone ; R₇, R₈, R₉ et R₁₀ sont réciproquement indépendants et représentent un substituant choisi parmi un atome d'hydrogène et un groupe alkyle avec de 1 à 3 atomes de carbone et deux substituants choisis parmi R₆, R₇, R₈, R₉ et R₁₀ peuvent être facultativement combinés ensemble et représentent un groupe alkylène avec de 1 à 5 atomes de carbone ;
à condition que, si deux substituants facultatifs quelconques choisis parmi R₆, R₇, R₈, R₉ et R₁₀ sont combinés ensemble et représentent un groupe alkylène avec de 1 à 5 atomes de carbone, les substituants différents des deux choisis sont réciproquement Indépendants et représentent un desdits substituants désignés pour le symbole;
R₁₁ représente un substituant choisi parmi un atome d'hydrogène, un groupe benzyle, un groupe p-hydroxybenzyle, un groupe cyclohexylméthyle, un groupe phényle, un groupe cyclohexyle, un groupe phényléthyle et un groupe cyclohexyléthyle, et R₁₂ représente un substituant choisi parmi un atome d'hydrogène et un groupe alkyle avec de 1 à 3 atomes de carbone, et R₁₃ représente un substituant choisi parmi des groupes alkyle ayant de 1 à 4 atomes de carbone.

2. Dérivé selon la revendication 1, dans lequel R₃ représente un groupe méthoxy, R₁, R₂, R₄, R₅, R₇, R₈, R₉, R₁₀ et R₁₂ représentent un atome d'hydrogène, R₆ et R₁₃ représentent un groupe méthyle et R₁₁ représente un groupe benzyle.

3. Dérivé selon la revendication 1, dans lequel R₂ représente un groupe méthoxy, R₃ représente un groupe hydroxyle, R₁, R₄, R₅, R₇, R₈, R₉, R₁₀ et R₁₂ représentent un atome d'hydrogène, R₆ et R₁₃ représentent un groupe méthyle et R₁₁ représente un groupe benzyle.

4. Dérivé selon la revendication 1, dans lequel R₂ représente un groupe hydroxyle, R₃ représente un groupe méthoxy, R₁, R₄, R₅, R₇, R₈, R₉, R₁₀ et R₁₂ représentent un atome d'hydrogène, R₆ et R₁₃ représentent un groupe méthyle et R₁₁ représente un groupe benzyle.

5. Dérivé selon la revendication 1, dans lequel R₂ représente un groupe méthoxyle, R₃ représente un groupe hydroxy, R₁, R₄, R₅, R₇, R₈, R₉, R₁₀ et R₁₂ représentent un atome d'hydrogène, R₆ et R₁₃ représentent un groupe méthyle et R₁₁ représente un groupe p-hydroxybenzyle.

6. Dérivé selon la revendication 1, dans lequel R₂ représente un groupe hydroxyle, R₃ représente un groupe méthoxy, R₁, R₄, R₅, R₇, R₈, R₉, R₁₀ et R₁₂ représentent un atome d'hydrogène, R₆ et R₁₃ représentent un groupe méthyle et R₁₁ représente un groupe cyclohexylméthyle.

7. Dérivé selon la revendication 1, dans lequel R₃ représente un groupe méthoxy, R₁, R₂, R₄, R₅, R₈, R₉, R₁₀ et R₁₂ représentent un atome d'hydrogène, R₆, R₇ et R₁₃ représentent un groupe méthyle et R₁₁ représente un groupe benzyle.

8. Dérivé selon la revendication 1, dans lequel R₃ représente un groupe hydroxyle, R₁, R₂, R₄, R₅, R₈, R₉, R₁₀ et R₁₂ représentent un atome d'hydrogène, R₆, R₇ et R₁₃ représentent un groupe méthyle et R₁₁ représente un groupe benzyle.

9. Dérivé selon la revendication 1, dans lequel R₂ représente un groupe méthoxy, R₃ représente un groupe hydroxyle, R₁, R₄, R₅, R₈, R₉, R₁₀ et R₁₂ représentent un atome d'hydrogène, R₆, R₇ et R₁₃ représentent un groupe méthyle et R₁₁ représente un groupe benzyle.

10. Dérivé selon la revendication 1, dans lequel R₂ représente un groupe hydroxyle, R₃ représente un groupe méthoxy, R₁, R₄, R₅, R₈, R₉, R₁₀ et R₁₂ représentent un atome d'hydrogène, R₆, R₇ et R₁₃ représentent un groupe méthyle et R₁₁ représente un groupe benzyle.

11. Dérivé selon la revendication 1, dans lequel R₂ représente un groupe méthyle, R₃ représente un groupe hydroxyle, R₁, R₄, R₅, R₇, R₈, R₉, R₁₀ et R₁₂ représentent un atome d'hydrogène, R₆ et R₁₃ représentent un groupe méthyle et R₁₁ représente un groupe benzyle.

12. Dérivé selon la revendication 1, dans lequel R₁ représente un groupe hydroxyle, R₃ représente un groupe méthoxy, R₂, R₄, R₅, R₈, R₉, R₁₀ et R₁₂ représentent un atome d'hydrogène, R₆, R₇ et R₁₃ représentent un groupe méthyle et R₁₁ représente un groupe benzyle.

13. Dérivé selon la revendication 1, dans lequel R₁ représente un groupe hydroxyle, R₃ représente un groupe méthyle, R₂, P₄, R₅, R₈, R₉, R₁₀ et R₁₂ représentent un atome d'hydrogène, R₆, R₇ et R₁₃ représentent un groupe méthyle et R₁₁représente un groupe benzyle.

14. Dérivé selon la revendication 1, dans lequel R₂ et R₃ sont combinés ensemble et représentent un groupe méthylènedioxy, R₁, R₄, R₅, R₈, R₉, R₁₀ et R₁₂ représentent un atome d'hydrogène, R₆, R₇ et R₁₃ représentent un groupe méthyle et R₁₁ représente un groupe benzyle.

15. Dérivé selon la revendication 1, dans lequel R₂ représente un groupe méthyle, R₃ représente un groupe méthoxy, R₁, R₄, R₅, R₈, R₉, R₁₀ et R₁₂ représentent un atome d'hydrogène, R₆, R₇ et R₁₃ représentent un groupe méthyle et R₁₁ représente un groupe benzyle.

16. Dérivé selon la revendication 1, dans lequel R₂ représente un groupe méthyle, R₃ représente un groupe hydroxyle, R₁, R₄, R₅, R₈, R₉, R₁₀ et R₁₂ représentent un atome d'hydrogène, R₆, R₇ et R₁₃ représentent un groupe méthyle et R₁₁ représente un groupe benzyle.

17. Dérivé selon la revendication 1, dans lequel R₂ représente un groupe hydroxyle, R₃ représente un groupe méthyle, R₁, R₄, R₅, R₈, R₉, R₁₀ et R₁₂ représentent un atome d'hydrogène, R₆, R₇ et R₁₃ représentent un groupe méthyle et R₁₁ représente un groupe benzyle.

18. Dérivé selon la revendication 1, dans lequel R₂ représente un groupe méthoxy, R₃ représente un groupe hydroxyle, R₁, R₄, R₅, R₈, R₉, R₁₀ et R₁₂ représentent un atome d'hydrogène, R₆ et R₇ sont combinés ensemble et représentent un groupe tétraméthylène, R₁₁ représente un groupe benzyle et R₁₃ représente un groupe méthyle.

19. Dérivé selon la revendication 1, dans lequel R₂ représente un groupe hydroxyle, R₃ représente un groupe méthoxy, R₁, R₄, R₅, R₈, R₉, R₁₀ et R₁₂ représentent un atome d'hydrogène, R₆ et R₇ représentent un groupe méthyle, R₁₁ représente un groupe benzyle et R₁₃ représente un groupe éthyle.

20. Dérivé selon la revendication 1, dans lequel R₂ représente un groupe hydroxyle, R₃ représente un groupe méthoxy, R₁, R₄, R₅, R₈, R₉ et R₁₀ représentent un atome d'hydrogène, R₆, R₇, R₁₂ et R₁₃ représentent un groupe méthyle et R₁₁ représente un groupe benzyle.

21. Dérivé selon la revendication 1, dans lequel R₂ et R₃ représentent un groupe hydroxyle, R₁, R₄, R₅, R₉, R₉, R₁₀ et R₁₂ représentent un atome d'hydrogène, R₆, R₇ et R₁₃ représentent un groupe méthyle et R₁₁ représente un groupe benzyle.

22. Dérivé selon l'une quelconque des revendications 1 à 6 et 11, dans lequel, si les substituants R₆ et R₇ sont différents l'un de l'autre, la configuration de l'atome de carbone auquel R₆ est lié dans ladite formule est dans l'état (R), (S) ou (RS).

23. Dérivé selon la revendication 1, dans lequel, si les substituants R₈ et R₉ sont différents l'un de l'autre, la configuration de l'atome de carbone auquel R₈ est lié dans ladite formule se trouve dans l'état (R), (S) ou (RS).

24. Dérivé selon la revendication 1, dans lequel, si R₁₀ représente un substituant différent d'un atome d'hydrogène, la configuration de l'atome de carbone auquel R₁₀ est lié dans ladite formule se trouve dans l'état (R), (S) ou (RS).

25. Agent édulcorant ou aliment édulcoré ou produit semblable comprenant le dérivé selon la revendication 1 comme constituant effectif, lequel peut facultativement contenir un véhiculeur ou un agent de gonflement pour les agents édulcorants.

26. Procédé pour communiquer un caractère sucré à un produit nécessitant un caractère sucré comprenant l'étape d'addition d'un dérivé selon la revendication 1 audit produit.

27. Procédé selon la revendication 26, dans lequel le produit nécessitant un caractère sucré est un aliment, une confiserie, un chewing-gum, une boisson, un produit pharmaceutique, un produit pour l'hygiène, un produit de toilette, un cosmétique ou un produit vétérinaire destiné aux animaux différents des êtres humains.

28. Procédé pour la fabrication d'un composé de la formule (1) selon la revendication 1, dans laquelle R₁₀ représente un atome d'hydrogène, ledit procédé comprenant une étape de réaction d'un aldéhyde représenté par la formule générale (2) : 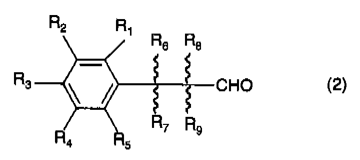 où R₁, R₂, R₃, R₄, R₅, R₆, R₇, R₈ et R₉ sont comme définis dans la formule (1) de la revendication 1 ;
avec un dérivé d'aspartame comme représenté par la formule générale (3) suivante: 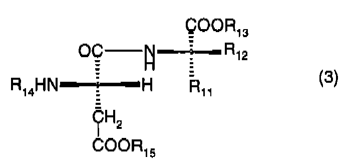 dans des conditions d'alkylation réductrice ;
où R₁₁, R₁₂ et R₁₃ dans la formule (3) sont comme définis dans la formule (1) de la revendication 1 ;
R₁₄ représente un atome d'hydrogène ou un substituant qui peut être converti en un atome d'hydrogène dans des conditions d'alkylation réductrice ; et
R₁₅ représente un atome d'hydrogène, un groupe benzyle ou un substituant qui peut être utilisé pour protéger un groupe carboxyle.

29. Procédé pour la fabrication d'un composé de la formule (1) selon la revendication 1, dans lequel R₇, R₉ et R₁₀ représentent un atome d'hydrogène, ledit procédé comprenant une étape de réaction d'un aldéhyde représenté par la formule (4) : 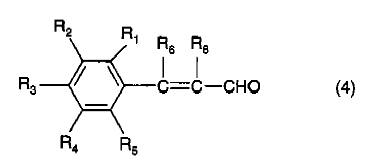 dans laquelle R₁, R₂, R₃, R₄, R₅, R₆ et R₈ sont comme définis dans la formule (1) de la revendication 1 ;
avec un dérivé d'aspartame comme représenté par la formule générale (3) suivante : 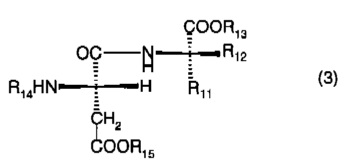 dans des conditions d'alkylation réductrice ;
où R₁₁, R₁₂ et R₁₃ dans la formule (3) sont comme définis dans la formule (1) de la revendication 1 ;
R₁₄ représente un atome d'hydrogène ou un substituant qui peut être converti en un atome d'hydrogène dans des conditions d'alkylation réductrice ; et
R₁₅ représente un atome d'hydrogène, un groupe benzyle ou un substituant qui peut être utilisé pour protéger un groupe carboxyle.

30. Procédé pour la fabrication d'un composé de la formule (1) selon la revendication 1 comprenant une étape de réaction d'un composé représenté par la formule générale (5) : 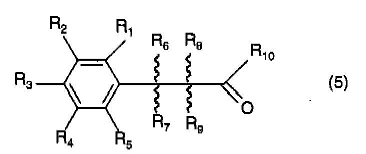 dans laquelle R₁, R₂, R₃, R₄, R₅, R₆, R₇, R₈, R₉ et R₁₀ sont comme définis dans la formule (1) de la revendication 1 ;
avec un dérivé d'aspartame comme représenté par la formule générale (3) suivante : 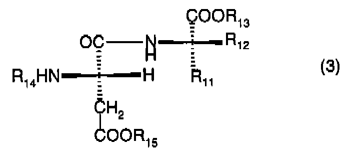 dans des conditions d'alkylation réductrice ;
où R₁₁, R₁₂ et R₁₃ dans la formule (3) sont comme définis dans la formule (1) de la revendication 1 ;
R₁₄ représente un atome d'hydrogène ou un substituant qui peut être converti en un atome d'hydrogène dans des conditions d'alkylation réductrice ; et
R₁₅ représente un atome d'hydrogène, un groupe benzyle ou un substituant qui peut être utilisé pour protéger un groupe carboxyle.
